(19) Europäisches Patentamt
European Patent Office
Office européen des brevets

(11) EP 2 776 447 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**05.01.2022 Patentblatt 2022/01**

(45) Hinweis auf die Patenterteilung:
**30.05.2018 Patentblatt 2018/22**

(21) Anmeldenummer: **12781343.4**

(22) Anmeldetag: **08.11.2012**

(51) Int Cl.:
*C07C 403/08* (2006.01) *C07C 403/06* (2006.01)
*C07F 9/54* (2006.01) *C07F 7/18* (2006.01)

(86) Internationale Anmeldenummer:
**PCT/EP2012/072144**

(87) Internationale Veröffentlichungsnummer:
**WO 2013/068465 (16.05.2013 Gazette 2013/20)**

(54) **VERFAHREN ZUR HERSTELLUNG VON OXOVINYLJONOL UND DESSEN O-GESCHÜTZTEN DERIVATEN**

METHOD FOR PRODUCING OXO-VINYL-IONOL AND O-PROTECTED DERIVATIVES THEREOF

PROCÉDÉ DE PRODUCTION D'OXOVINYLIONOL ET DE SES DÉRIVÉS O-PROTÉGÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2011 EP 11188401**

(43) Veröffentlichungstag der Anmeldung:
**17.09.2014 Patentblatt 2014/38**

(73) Patentinhaber: **BASF SE**
**67056 Ludwigshafen am Rhein (DE)**

(72) Erfinder:
- **ERNST, Hansgeorg**
  **67346 Speyer (DE)**
- **PUHL, Michael**
  **69493 Hirschberg (DE)**
- **BENSON, Stefan**
  **64646 Heppenheim (DE)**
- **SIEGEL, Wolfgang**
  **67117 Limburgerhof (DE)**

(74) Vertreter: **BASF IP Association**
**BASF SE**
**G-FLP-C006**
**67056 Ludwigshafen (DE)**

(56) Entgegenhaltungen:

| | |
|---|---|
| **EP-A2- 0 101 597** | **EP-A2- 0 198 351** |
| **WO-A1-2008/145627** | **WO-A2-2007/072529** |
| **CN-A- 1 817 842** | **CN-A- 1 978 418** |
| **CN-A- 101 723 769** | **DE-A1- 2 212 948** |
| **DE-A1- 2 537 072** | **DE-A1- 2 704 406** |
| **DE-C2- 2 801 908** | **US-A- 4 098 827** |

- **ROSENBERGER M ET AL: "CANTHAXANTHIN. A NEW TOTAL SYNTHESIS", JOURNAL OF ORGANIC CHEMISTRY, ACS, US, Bd. 47, Nr. 11, 1. Januar 1982 (1982-01-01), Seiten 2130-2134, XP002230683, ISSN: 0022-3263, DOI: 10.1021/JO00132A028**
- **ROTHENBERG ET AL: "Copper-catalyzed homolytic benzylic and allylic oxidation using tert-butyl hydroperoxide", JOURNAL OF THE CHEMICAL SOCIETY, PERKIN TRANSACTIONS 2, CHEMICAL SOCIETY. LETCHWORTH, GB, 1. Januar 1998 (1998-01-01), Seiten 2429-2434, XP002300005, ISSN: 1472-779X, DOI: 10.1039/A805324C**
- **HANSGEORG ERNST: "Recent advances in industrial carotenoid synthesis", PURE AND APPLIED CHEMISTRY, Bd. 74, Nr. 11, 1. Januar 2002 (2002-01-01), Seiten 2213-2226, XP055046436, ISSN: 0033-4545, DOI: 10.1351/pac200274112213 in der Anmeldung erwähnt**
- **ARTHUR J. CATINO et al.: "Dirhodium(II) Caprolactamate: An Exceptional Catalyst for Allylic Oxidation", Journal of American Chemical Society, vol. 126, 2004, pages 13622-13623,**
- **MAGDALENA JURADO-GONZALEZ et al.: "Allylic and benzylic oxidation using cobalt(II) alkyl phosphonate modified silica", Tetrahedron Letters, vol. 44, no. 22, 2003, pages 4283-4286,**
- **TONY K. M. SHING et al.: "Mild Manganese(III) Acetate Catalyzed Allylic Oxidation: Application to Simple and Complex Alkenes", Organic Letters, vol. 8, no. 14, 2006, pages 3149-3151,**



Printed by Jouve, 75001 PARIS (FR)

**(Forts. nächste Seite)**

- JORGE A. R. SALVADOR et al.: "The allylic oxidation of unsaturated steroids by tert-butyl hydroperoxide using homogeneous and heterogeneous cobalt acetate", Chem. Commun., 2001, pages 33-34,
- K. SRINIVASAN et al.: "Dual Pathways for Manganese Catalysis of Olefin Oxidation with alkyl hydroperoxides", Journal of Molecular Catalysis, vol. 36, no. 3, 1986, pages 297-317,
- GEORGE A. OLAH et al.: In: "Hydrocarbon Chemistry", 2003, WILEY-INTERSCIENCE pages 449-488, 522-528,
- Dafeng Y. et al.. Chinese Journal of Synthetic Chemistry. Vol. 16. No. 4 (2008). 418-422
- ELISABETH BECHER et al.: "SYNTHESE VON ASTAXANTHIN AUS BETA-JONON. I. ERSCHLIESSUNG DER ENANTIOMEREN C15-WITTIGSALZE DURCH CHEMISCHE UND MIKROBIOLOGISCHE RACEMATSPALTUNG VON (PLUS OR MINUS)-3-ACETOXY-4-OXO-BETA-JONON", Helvetica Chimica Acta, vol. 64, no. 7, 1981, pages 2419-2435,
- Kienzle, F.: Helvetica Chimica Acta 58(1) (1975). S. 27-40

## Beschreibung

Die vorliegende Erfindung betrifft die Herstellung von Oxovinyljonol und dessen O-geschützten Derivaten der Formel I

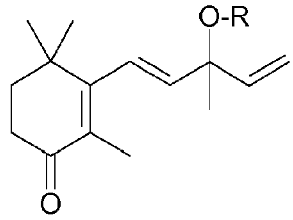

(I)

worin R für Wasserstoff oder eine OH-Schutzgruppe, beispielsweise eine Gruppe $Si(R^a)_3$ steht, worin die Reste $R^a$ gleich oder verschieden sein können und unabhängig voneinander für $C_1$-$C_4$-Alkyl stehen. Die Erfindung betrifft auch die Verwendung von Verbindungen der Formel I in der Herstellung von Astaxanthin oder Canthaxanthin und Vorstufen davon, insbesondere von Phosphoniumsalzen der Formel IV

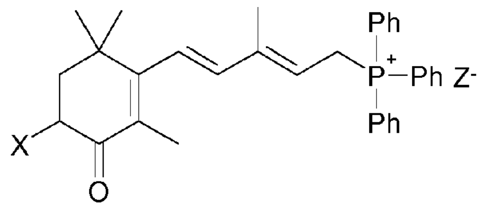

(IV)

worin X für Wasserstoff, OH oder OR" steht, Ph für Phenyl steht, Z- ein Halogenid-Anion, insbesondere ein Bromid-Anion, bedeutet und R" für Wasserstoff oder eine OH-Schutzgruppe steht.

Xanthophylle wie Astaxanthin und Canthaxanthin sind wertvolle Futtermittelzusatzstoffe. Beispielsweise wird Astaxanthin dem Fischmehl für Zuchtlachs zugesetzt um die charakteristische Fleischfarbe zu erhalten. Canthaxanthin wird direkt zum Nachfärben von Seelachs verwendet oder Hühnerfutter zugemengt, um die Farbe des Eigelbs zu intensivieren. Zentraler Schritt der technischen Synthese dieser Verbindung ist die Umsetzung eines Phosphoniumsalzes der Formel IV mit dem C10-Dialdehyd (2E,4E,6E)-2,7-Dimethylocta-2,4,6-triendial im Sinne einer doppelten Wittig-Olefininierung (siehe Pure Appl. Chem., 74 (2002) 2213 ff.).

In der Literatur werden verschiedene Verfahren zur Herstellung von Phosphoniumsalzen der Formel IV beschrieben, die von petrochemischen Basischemikalien ausgehen. Eine Übersicht findet sich beispielsweise in "Carotenoids", Band 2 - Synthesis, S. 281-284; Birkhäuser, 1996 sowie in Pure Appl. Chem., 74 (2002) 2213 ff.. Diese Verfahren sind vielstufig, was zu niedrigen Gesamtausbeuten sowie hohem Ressourcenverbrauch und damit letztlich zu hohen Herstellkosten führt.

WO 2007/072529 beschreibt ein Verfahren zur Herstellung von Phosphoniumsalzen der Formel IV, worin X für OH steht und Z- ein Bromidion bedeutet, in 11 Stufen, ausgehend von gut verfügbarem β-Ionon, umfassend die Oxidation von β-Ionon zum 4-Oxo-β-ionon unter Verwendung von (Erd)alkalimetallbromiden in Gegenwart von Iod oder (Erd)alkalimetalliodiden in einem sauren Reaktionsmedium.

EP 101597 beschreibt ein Verfahren zur Herstellung von Phosphoniumsalzen der Formel IV, das von Oxovinyljonol der Formel I, (R = Wasserstoff) ausgeht und die Verbindung IV in nur vier Stufen liefert. Dieses Verfahren hatte jedoch den Nachteil, dass bislang Oxovinyljonol seinerseits nur in einer mehrstufigen Synthese zugänglich war. So beschreiben J. Org. Chem., 47 (1982), 2130-2134 und US 4,098, 827 die Herstellung von Oxovinyljonol aus leicht verfügbarem α-Jonon in einer vierstufigen Sequenz. Auch dieser Zugang zu der Verbindung IV bedeutet damit letztlich eine vielstufige Reaktionssequenz, verbunden mit niedrigen Gesamtausbeuten, hohem Ressourcenverbrauch und hohen Herstellkosten.

WO 2008/145627 beschreibt ausschließlich ein Verfahren zur elektrochemischen Oxidation von Kohlenstoffatomen, die sich in α-Stellung zur Doppelbindung befinden, unter Verwendung von Mediatoren, wobei die Mediatoren Redoxpaare sind, die eine elektrochemische Oxidation ermöglichen.

CN 101723769 beschreibt kursorisch ein katalytisches Verfahren zur Oxidation von Iononen-Verbindungen. Diese Verbindungen weisen in der Seitenkette keine Vinylgruppe auf.

Der vorliegenden Erfindung liegt daher die Aufgabe zugrunde, ein Verfahren zur Herstellung von Oxovinyljonol (Formel I, R = Wasserstoff) oder seiner O-geschützten Derivate (Formel I, R = OH-Schutzgruppe) bereitzustellen, das

ausgehend von gut verfügbaren Ausgangsverbindungen die Herstellung der Verbindungen I in einfacher Weise und mit wenigen Stufen erlaubt.

Es wurde überraschenderweise gefunden, dass diese Aufgabe dadurch gelöst wird, dass man die gut verfügbare Verbindung der Formel II,

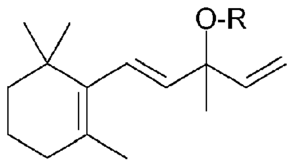
(II)

worin R die zuvor für Formel I angegebenen Bedeutungen aufweist, d.h. β-Vinyljonol (Formel II, R = Wasserstoff) oder ein O-geschütztes Derivat davon (Formel II, R = OH-Schutzgruppe) mit einem Oxidationsmittel in Gegenwart wenigstens eines Übergangsmetalls umsetzt, welches ausgewählt ist unter Cu, Co, Fe, Mn und deren Mischungen und wobei das Oxidationsmittels wenigstens eine sauerstoffhaltige Verbindung umfasst, die unter organischen Hydroperoxiden ausgewählt ist.

Dementsprechend betrifft die vorliegende Erfindung ein Verfahren zur Herstellung von Oxovinyljonol und dessen O-geschützten Derivaten der zuvor definierten Formel I, das dadurch gekennzeichnet ist, dass man β-Vinyljonol oder ein O-geschütztes Derivat davon der Formel II, worin R die zuvor genannten Bedeutungen aufweist, mit einem Oxidationsmittel in Gegenwart eines oder mehrerer Übergangsmetalle umsetzt, wobei das Oxidationsmittels wenigstens eine sauerstoffhaltige Verbindung umfasst, die unter organischen Hydroperoxiden ausgewählt ist.

Ausgehend von dem als etabliertes Vitamin-A-Vorprodukt gut und in großen Mengen verfügbaren β-Vinyljonol und seinen O-geschützten Derivaten liefert das erfindungsgemäße Verfahren Oxovinyljonol bzw. seine O-geschützten Derivate in nur einem Reaktionsschritt in guten Ausbeuten.

Auf diese Weise vereinfacht sich insbesondere auch der Zugang zu den Verbindungen der Formel IV, insbesondere solchen Verbindungen der Formel IV, worin X für OH steht und Z- ein Bromidion bedeutet. Dementsprechend betrifft die vorliegende Erfindung auch ein Verfahren zur Herstellung von Verbindungen der Formel IV

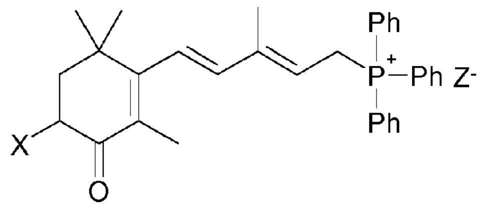
(IV)

worin X für Wasserstoff, OH oder OR" steht, Ph für Phenyl steht, Z- ein Halogenid-Anion bedeutet und R" für Wasserstoff oder eine OH-Schutzgruppe steht, umfassend:

1. i) Bereitstellung von Oxovinyljonol oder eines O-geschützten Derivats davon der Formel I durch das erfindungsgemäße Verfahren wie hier und im Folgenden beschrieben;
2. ii) Umwandlung von Oxovinyljonol oder seines O-geschützten Derivats der Formel I in an sich bekannter Weise in eine Verbindung der Formel IV, z.B. nach dem Verfahren der EP 101597, oder nach den hier und im Folgenden beschriebenen Methoden.

In den Formeln I und II steht R für Wasserstoff oder eine OH-Schutzgruppe. Geeignete OH-Schutzgruppen sind dem Fachmann bekannt, z.B. aus P. J. Kocienski, Protecting Groups, Kapitel 2, Georg-Thieme-Verlag, Stuttgart 2000 oder P.G.M. Wuts et al. Greene's Protective Groups in Organic Synthesis, 4. Auflage, John-Wiley & Xons, 2006. Hierzu zählen beispielsweise Gruppen, in denen die Gruppe OR Bestandteil einer Ethergruppe, Silylethergruppe, Acetalschutzgruppe, Carbonsäureestergruppe oder Carbonatgruppe ist. Geeignete Ethergruppen OR sind insbesondere Benzyl-, Trityl-, Alkyl- und Allylethergruppen, d.h. R steht für gegebenenfalls substituiertes Benzyl, z.B. Benzyl, 4-Methoxybenzyl oder 3,4-Dimethoxybenzyl, Triphenylmethyl (Trityl), $C_1$-$C_4$-Alkyl wie Methyl, Ethyl oder tert-Butyl, Allyl (2-Propenyl) oder 2-Methoxy-2-propenyl. Geeignete Silylethergruppen OR sind insbesondere solche, in denen R für einen Rest der Formel $SiR^aR^bR^c$ steht, worin $R^a$ für $C_1$-$C_4$-Alkyl, z.B. Methyl, Ethyl, Propyl, Isopropyl, n-Butyl oder tert.-Butyl steht, $R^b$ und $R^c$ gleich oder verschieden sind und unabhängig voneinander für $C_1$-$C_4$-Alkyl, wie zuvor definiert, Cycloalkyl, z.B. Cyclopentyl oder Cyclohexyl, oder Phenyl stehen. Beispiele für Reste der Formel $SiR^aR^bR^c$ sind insbesondere Trimethylsilyl, Triethylsilyl, Triisopropylsilyl, tert.Butyldimethylsilyl, tert.Butyldiphenylsilyl und Diethylisopropyl. Geeignete Acetalschutzgruppen sind insbesondere solche, in denen die Gruppe OR Bestandteil eines Formaldehyd-Acetals ist, z.B. Gruppen, in denen R für Methoxymethyl, 1-Methoxy-1-methylethyl, 1-Ethoxyethyl, Methylthiomethyl, (2-Methoxyethoxy)methyl, Benzyloxymethyl, β-(Trimethylsilyl)-ethoxymethyl steht. Ein Beispiel für eine Acetalschutzgruppe ist weiterhin der 2-Tetrahydropyranyl Rest, d.h. R steht für 2-Tetrahydropyranyl. Geeignete Carbonsäureestergruppen OR sind insbesondere solche, in denen R für Acetyl, Chloracetyl, Pivaloyl oder Benzoyl steht. Geeignete Carbonatgruppen OR sind insbesondere solche, in denen R für einen Rest der Formel C(O)-$OR^x$ steht, worin $R^x$ für $C_1$-$C_4$-Alkyl, z.B. tert.-Butyl, oder für Allyl, Benzyl oder Flouren-9-ylmethyl steht.

Vorzugsweise steht R in den Formeln I und II für Wasserstoff. Sofern R für einen von Wasserstoff verschiedenen Rest steht, dann steht es vorzugsweise für einen Rest der Formel $SiR^aR^bR^c$ steht, worin $R^a$, $R^b$ und $R^c$ die zuvor genannten Bedeutungen aufweisen und worin $R^b$ und $R^c$ insbesondere für $C_1$-$C_4$-Alkyl stehen.

Erfindungsgemäß setzt man eine Verbindung der Formel II, worin R die zuvor genannten Bedeutungen aufweist und insbesondere für Wasserstoff steht, mit einem Oxidationsmittel in Gegenwart eines Übergangsmetalls, wie zuvor genannt, um, wobei das Oxidationsmittels wenigstens eine sauerstoffhaltige Verbindung umfasst, die unter organischen Hydroperoxiden ausgewählt ist.

Bevorzugt umfasst das Oxidationsmittel wenigstens ein organisches Hydroperoxid. Beispiele für organische Hydroperoxide sind Alkylhydroperoxide und Aralkylhydroperoxide, insbesondere tertiäre $C_4$-$C_8$-Alkylhydroperoxide wie tert. Butylhydroperoxid und tert.-Amylperoxid, sowie tertiäre, ggf. am Phenylring durch 1, 2 oder 3 $C_1$-$C_4$-Alkylgruppen substituierte Phenyl-$C_3$-$C_8$-alkylhydroperoxide wie Cumolhydroperoxid. Bevorzugte organische Hydroperoxide sind Alkylhydroperoxide, insbesondere tertiäre $C_4$-$C_8$-Alkylhydroperoxide, speziell tert. Butylhydroperoxid und tert.-Amylperoxid, und deren Gemische.

Als Oxidationsmittel kann die sauerstoffhaltige Verbindung alleine oder in Kombination mit einem weiteren Oxidationsmittel eingesetzt werden, wobei das weitere Oxidationsmittel vorzugsweise ausgewählt ist aus der Gruppe der Alkali- oder Erdalkalimetallhalogenite oder -hypohalogenite, insbesondere aus der Gruppe der Alkalimetall- und Erdalkalimetallchlorite und -hypochlorite wie Natriumchlorit oder Natriumhypochlorit.

In einer Ausführungsform der Erfindung wird die unter organischen Hydroperoxiden ausgewählte sauerstoffhaltige Verbindung als alleiniges Oxidationsmittel eingesetzt. In einer bevorzugten Ausführungsform der Erfindung wird wenigstens ein unter Alkylhydroperoxiden und Aralkylhydroperoxiden, insbesondere unter tertiären $C_4$-$C_8$-Alkylhydroperoxiden wie tert. Butylhydroperoxid und tert.-Amylperoxid, und tertiären Phenyl-$C_3$-$C_8$-alkylhydroperoxiden wie Cumolhydroperoxid ausgewähltes organisches Hydroperoxid als alleiniges Oxidationsmittel ausgewählt.

In der Regel wird man das Oxidationsmittel in wenigstens äquimolarer Menge einsetzen, d.h. in einer Menge von wenigstens 1 mol pro mol der Verbindung der Formel II, vorzugsweise in einer Menge von wenigstens 2 mol pro mol der Verbindung der Formel I, z.B. in einer Menge von 1 bis 10 mol, insbesondere 2 bis 7 mol und speziell 2 bis 4 mol pro mol der Verbindung der Formel II. Dies gilt insbesondere, wenn die unter organischen Hydroperoxiden ausgewählte sauerstoffhaltige Verbindung alleiniges Oxidationsmittel ist. Sofern eine Kombination aus sauerstoffhaltiger Verbindung und weiterem Oxidationsmittel eingesetzt wird, beziehen sich diese Angaben auf die Gesamtmenge der eingesetzten Äquivalente an Oxidationsmittel, wobei 1 Mol Halogenit 2 Mol der sauerstoffhaltigen Verbindung entspricht und 1 Mol Hypohalogenit 1 Mol der sauerstoffhaltigen Verbindung entspricht.

Erfindungsgemäß setzt man eine Verbindung der Formel II, worin R die zuvor genannten Bedeutungen aufweist und insbesondere für Wasserstoff steht, mit einem Oxidationsmittel in Gegenwart wenigstens eines Übergangsmetalls, wie zuvor genannt, z.B. in Gegenwart von einem Übergangsmetall oder in Gegenwart einer Kombination von 2 oder 3 verschiedenen Übergangsmetallen um. Das wenigstens eine Übergangsmetall kann dabei in elementarer Form oder in Form einer chemischen Verbindung, z.B. in Form eines Salzes, in Form eines Oxids oder in Form einer Komplexverbindung oder einer Mischung davon, eingesetzt werden. Bevorzugt wird das wenigstens eine Übergangsmetall in Form einer oder mehrerer Übergangsmetallverbindungen, insbesondere in Form eines oder mehrerer Salze und/oder einer oder mehrerer Komplexverbindungen eingesetzt.

Typischerweise setzt man das wenigstens eine Übergangsmetall in einer Gesamtmenge von $5x10^{-6}$ bis 0,5 mol, insbesondere in einer Menge von $5x10^{-5}$ bis 0,3 mol und speziell in einer Menge von $5x10^{-4}$ bis 0,3 mol pro mol der Verbindung der Formel II ein.

Erfindungsgemäß sind Übergangsmetalle ausgewählt unter Cu, Fe, Co oder Mn und deren Mischungen.

Sofern das Übergangsmetall als Salz eingesetzt wird, sind geeignete Gegenionen beispielsweise Halogenid wie Chlorid, Bromid oder Jodid, Pseudohalogenide wie Cyanid, Tetrafluoroborat, Tetrafluorophosphat, Anionen von organischen Monocarbonsäuren, z.B. $C_1$-$C_{18}$-Alkanoat (= $C_1$-$C_{18}$-Alkancarboxylat), das chloriert oder fluoriert sein kann und/oder worin eine $CH_2$-$CH_2$-Gruppe durch eine $C_3$-$C_6$-Cycloalkandiylgruppe ersetzt sein kann, wie Formiat, Acetat, Propionat, Octanoat, die Isomeren des Ethylhexanoat, Naphthenat (= Anion der Naphthensäure: Gemisch alkylierter Cycloalkancarbonsäuren CAS-Nr. 1338-24-5), Chloracetat, Dichloracetat, Trichloracetat, Trifluoracetat, Anionen von Dicarbonsäuren wie Oxalat, $C_1$-$C_4$-Alkylsulfonat, das chloriert oder fluoriert sein kann, wie Methansulfonat oder Trifluormethansulfonat, Arylsulfonat wie Phenylsulfonat oder Toluolsulfonat, Sulfat, Hydrogensulfat und Triflate.

Wenn das Übergangsmetall als Komplexverbindung eingesetzt wird, sind geeignete Liganden beispielsweise

Acetylacetonat (acac), Pyridin, Alkylpyridine, Pyrazol, Alkylpyrazole, Imidazol, Trispyridylmethyamin der Formel A

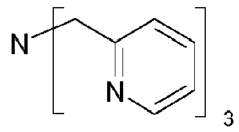

(A)

sowie Liganden vom Salentyp, der durch die folgende Formel B (dargestellt in der protonierten, freien Ligand-Form) repräsentiert wird,

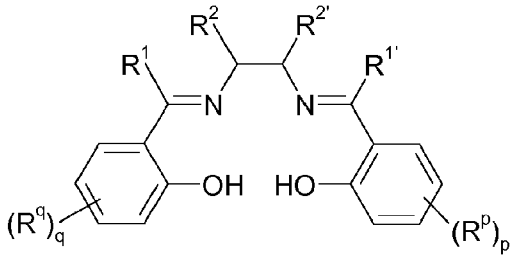

(B)

worin $R^1$ und $R^{1'}$ unabhängig voneinander für Wasserstoff oder $C_1$-$C_4$-Alkyl stehen, $R^2$ und $R^{2'}$ unabhängig voneinander für Wasserstoff oder $C_1$-$C_4$-Alkyl stehen oder gemeinsam für Butan-1,4-diyl stehen, $R^p$, $R^q$ unabhängig voneinander für Halogen, COOH, $SO_3H$, $C_1$-$C_4$-Alkyl, $C_1$-$C_4$-Alkoxy oder ggf. substituiertes Phenyl stehen und p und q unabhängig voneinander für 0, 1, 2, 3 oder 4 stehen und insbesondere 0, 1 oder 2 bedeuten, wobei im Falle von p bzw. q = 2, 3 oder 4 die Reste $R^p$ und $R^q$ jeweils gleich oder verschieden sein können. Beispiele für Liganden der Formel B sind: N,N'-Bis(salicyliden)ethylendiamin (im Folgenden Salen-B1) und N,N'-Bis(salicyliden)cyclohexan-1,2-diamin (im Folgenden Salen-B2).

Geeignete Liganden sind weiterhin Verbindungen, die wenigstens eine Oxazolgruppe aufweisen, z.B. Oxazol, Verbindungen mit einer 1,10-Phenanthrolin-Gruppe, z.B. 1,10-Phenanthrolin und substituierte 1,10-Phenanthroline, substituierte und unsubstituierte Phthalocyanine sowie Verbindungen mit einer 2,2-Bipyridingruppe, z.B. 2,2-Bipyridin und substituierte 2,2-Bipyridine. In Zusammenhang mit 1,10 Phenanthrolin bedeutet "ggf. substituiert", dass ein oder mehrere Wasserstoffatome, beispielsweise 1, 2, 3 oder 4 Wasserstoffatome des 1,10-Phenanthrolins, vorzugsweise die Wasserstoffatome der 3-, 4-, 5-, 6-, 7- oder 8-Position, durch Substituentengruppen ersetzt sind, wobei die Substituenten beispielsweise ausgewählt sind unter Halogen, COOH, $SO_3H$, $C_1$-$C_4$-Alkyl, $C_1$-$C_4$-Alkoxy, CN und ggf. substituiertem Phenyl. In Zusammenhang mit 2,2-Bipyridin bedeutet "ggf. substituiert", dass ein oder mehrere Wasserstoffatome, beispielsweise 1, 2, 3 oder 4 Wasserstoffatome des 2,2-Bipyridins, vorzugsweise die Wasserstoffatome der 4-, 4'-, 5- oder 5'-Position, durch Substituentengruppen ersetzt sind, wobei die Substituenten beispielsweise ausgewählt sind unter Halogen, COOH, $SO_3H$, $C_1$-$C_4$-Alkyl, $C_1$-$C_4$-Alkoxy, CN und ggf. substituiertem Phenyl. In Zusammenhang mit Phthalocyanin bedeutet "substituiert", dass ein oder mehrere Wasserstoffatome, beispielsweise 1 bis 8 Wasserstoffatome des Phthalocyanins durch Substituentengruppen ersetzt sind, wobei die Substituenten beispielsweise ausgewählt sind unter Halogen, COOH, $SO_3H$, $C_1$-$C_4$-Alkyl, $C_1$-$C_4$-Alkoxy, CN und ggf. substituiertem Phenyl. Gegebenenfalls substituiertes Phenyl bedeutet, dass die Phenylgruppe 1, 2, 3 oder 4 Substituenten tragen kann, die beispielsweise ausgewählt sind unter Halogen, COOH, $SO_3H$, $C_1$-$C_4$-Alkyl, $C_1$-$C_4$-Alkoxy und CN.

Geeignet sind weiterhin Liganden der allgemeinen Formel C (dargestellt in der protonierten, freien Ligand-Form)

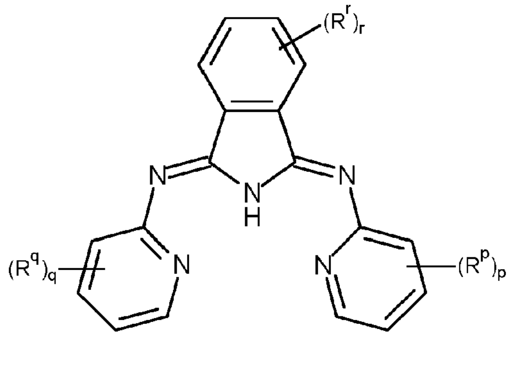

(C)

worin p, q, $R^p$, $R^q$ die zuvor für Formel B genannten Bedeutungen aufweisen, r für 0, 1, 2 oder 3 steht und insbesondere 0 oder 1 bedeutet und $R^r$ für Halogen, $C_1$-$C_4$-Alkyl, oder $C_1$-$C_4$-Alkoxy steht, wobei im Falle von r = 2 oder 3 $R^r$ gleich oder verschieden sein kann. Derartige Liganden und Komplexe davon mit Übergangsmetallverbindungen sind bekannt, z.B. aus Z. Anorg. Chem, 2004, 630, S. 1962. Ein Beispiel für einen bevorzugten Liganden der Formel C ist die Verbindung C1, worin p, q und r in Formel C für 0 stehen.

Sofern die Übergangsmetalle in Form von Komplexverbindungen eingesetzt werden, weist die Komplexverbindung typischerweise so viele Liganden auf, dass in der Komplexverbindung das Molverhältnis aller Koordinationsstellen der Liganden, welche das Metallatom in der Komplexverbindung koordinieren, typischerweise 1:1 bis 1:6 beträgt. Bei einzähnigen Liganden (eine Koordinationsstelle im Ligand) beträgt das Molverhältnis von Übergangsmetall zu Ligand daher typischerweise 1:1 bis 1:6 , bei zweizähnigen Liganden (zwei Koordinationsstellen im Ligand) typischerweise 1:1 bis 3:1 und bei 3- oder höherzähnigen Liganden, z.B. 3- oder 4-zähnigen Liganden typischerweise 1:1 bis 1,1:1. Es können auch Komplexverbindungen eingesetzt werden, die Liganden mit unterschiedlicher Zähnigkeit aufweisen, z.B. einen oder mehrere einzähnige Liganden und einen oder mehrere zweizähnige Liganden oder einen oder mehrere einzähnige Liganden und einen höherzähnigen, z.B. 3- oder 4-zähnigen Liganden.

Die Komplexverbindungen der Übergangsmetalle können salzartig sein, d.h. die gegebenenfalls durch den bzw. die Liganden eingebrachte negative Ladung reicht nicht zur Kompensation der positiven Ladung des Übergangsmetalls aus. In diesen Fällen weisen die Komplexverbindungen ein oder mehrere der vorgenannten Anionen als Gegenionen auf.

Das Übergangsmetall kann auch in elementarer Form eingesetzt werden. Häufig wird man es dann in geträgerter Form, z.B. auf einem unter Aluminiumoxid, Siliziumdioxid oder Kohlenstoff ausgewählten Trägermaterial, einsetzen. Das Übergangsmetall kann auch in Form eines Oxides oder Gemisches von Oxiden eingesetzt werden, z.B. $Cu_2O$, $Co_2O_3$, oder in Form eines Mischoxids, z.B. als Mischoxide z.B. Aluminium oder Nickel.

Sofern man das Übergangsmetall in Form eines Salzes, einer Komplexverbindung, eines Oxids, eines Mischoxids oder in elementarer Form einsetzt, kann man das erfindungsgemäße Verfahren auch in Gegenwart eines oder mehrerer der im Zusammenhang mit den Übergangsmetallkomplexverbindungen genannten Liganden durchführen. Sofern der Ligand flüssig ist, beispielsweise im Falle des Pyridins, kann man die Umsetzung in dem Liganden als Lösung- bzw. Verdünnungsmittel durchführen.

In einer bevorzugten Ausführungsform erfolgt die Umsetzung der Verbindung der Formel II mit dem Oxidationsmittel in Gegenwart wenigstens eines Liganden, der zur Ausbildung einer oder mehrerer koordinativer Bindungen mit dem Übergangsmetall geeignet ist. Hierzu kann der Ligand in Form der Übergangsmetallkomplexverbindung eingesetzt werden, die den Liganden bereits enthält, oder separat von dem Übergangsmetall zum Reaktionsansatz gegeben, wobei sich vermutlich unter den Reaktionsbedingungen eine Komplexverbindung ausbildet, welche das Übergangsmetall und den Liganden enthält.

Für diesen Zweck sind insbesondere solche Liganden bevorzugt, die wenigstens ein Stickstoffatom enthalten. Beispiele für derartige Liganden sind Pyridin, Alkylpyridine, Pyrazol, Alkylpyrazole, Imidazol, Trispyridylmethylamine der Formel A, Liganden vom Salentyp der Formel B, Liganden der Formel C, Verbindungen, die wenigstens eine Oxazolgruppe aufweisen, z.B. Oxazol, Verbindungen mit einer 1,10-Phenanthrolin-Gruppe, z.B. 1,10-Phenanthrolin und substituierte 1,10-Phenanthroline, substituierte und unsubstituierte Phthalocyanine sowie Verbindungen mit einer 2,2-Bipyridingruppe. Für diesen Zweck sind solche Liganden besonders bevorzugt, worin das wenigstens eine Stickstoffatom in Form einer Oxazol-, 2,2-Bipyridin- und 1,10-Phenanthrolingruppe vorliegt.

Sofern man die Umsetzung der Verbindung der Formel II mit dem Oxidationsmittel in Gegenwart wenigstens eines Liganden durchführt, der zur Ausbildung einer oder mehrerer koordinativer Bindungen mit dem Übergangsmetall geeignet ist, insbesondere in Gegenwart wenigstens eines Liganden, der wenigstens ein Stickstoffatom enthält, und speziell wenigstens eines Liganden, aus der Gruppe, Pyridin, Bipyridin-Verbindungen, Phenanthrolin-Verbindungen, Oxazolverbindungen und Liganden der allgemeinen Formeln A, B und C, ganz speziell aus der Gruppe der Lignanden, die eine Oxazol-, 2,2-Bipyridin- und 1,10-Phenanthrolingruppe aufweisen, liegt das Molverhältnis von Metall zu Ligand vorzugsweise im Bereich von 0,5 : 1 bis 2:1.

Insbesondere setzt man das Übergangsmetall in Form wenigstens einer Verbindung ein, die unter den Halogeniden, Acetylacetonaten, Oxalaten und Salzen organischer Monocarbonsäuren, wie Naphthenaten oder $C_2$-$C_{18}$-Alkanoaten, des Kupfers, des Kobalts oder des Mangans und den N,N-Salicyliminoethankomplexen des Kupfers, des Kobalts oder des Mangans ausgewählt ist, und die speziell unter CuCl, $CuCl_2$, CuI, $CoCl_2$, Cu(II)-Oxalat, Co(II)-Salzen von organischen Monocarbonsäuren (z.B. von Napthenaten oder $C_1$-$C_{18}$-Alkanoaten), wie $Co(Formiat)_2$, $Co(Acetat)_2$, $Co(2\text{-}Ethylhexanoat)_2$ oder $Co(Naphthenat)_2$, Co(II)-Oxalat, $Co(acac)_2$, $Co(acac)_3$, Co(salen), Co(salen)Cl, Mn(salen) und Mn(salen)Cl, und $Mn(acac)_2$ ausgewählt sind, wobei acac für Acetylacetonat steht und salen für den N,N'-Bis(salicyliden)ethylendiamino-Liganden B1 steht. Die vorgenannten Übergangsmetallverbindungen können dabei als solche oder in Gegenwart weiterer Liganden, speziell in Gegenwart wenigstens eines Liganden aus der Gruppe Bipyridin-Verbindungen, Phenanthrolin-Verbindungen und Oxazolverbindungen und ganz speziell in Gegenwart eines Phenanthrolin-Liganden eingesetzt werden. In dieser ganz speziellen Ausführungsform liegt das Molverhältnis von Metall zu

Ligand vorzugsweise im Bereich von 0,5 : 1 bis 2:1.

In einer speziellen Ausführungsform setzt man wenigstens eine Übergangsmetallverbindung ein, die unter Verbindungen des Kobalts, insbesondere unter Acetylacetonaten, Naphthenaten und $C_1$-$C_{18}$-Alkanoaten des Kobalts und N,N-Salicyliminoethan-komplexen des Kobalts ausgewählt ist, z.B. Kobaltbisacetylacetonat, Kobalttrisacetylacetonat, Kobalt(II)-2-ethylhexanoat, Kobalt(II)acetat, Kobalt(II)oxalat, Kobalt(II)-naphthenat, Kobalt(II)salicyliminoethan, Chlorokobalt(III)salicyliminoethan. Vorzugsweise führt man in dieser speziellen Ausführungsform die Umsetzung in Gegenwart wenigstens eines weiteren Liganden, insbesondere in Gegenwart wenigstens eines Liganden aus der Gruppe Bipyridin-Verbindungen, Phenanthrolin-Verbindungen und Oxazolverbindungen und ganz speziell in Gegenwart eines Phenanthrolin-Liganden durch. In dieser ganz speziellen Ausführungsform liegt das Molverhältnis von Metall zu Ligand vorzugsweise im Bereich von 0,5 : 1 bis 2:1.

In einer weiteren speziellen Ausführungsform des erfindungsgemäßen Verfahrens setzt man als Übergangsmetall wenigstens ein Salz des Kupfers, des Kobalts, des Eisens oder des Mangans oder eine Mischung davon ein, das insbesondere unter den Halogeniden, Acetylacetonaten (acac), Oxalaten, Salzen organischer Monocarbonsäuren wie Naphthenaten oder $C_2$-$C_{18}$-Alkanoaten des Kupfers, des Kobalts oder Mangans sowie den Komplexen des N,N-Salicyliminoethans (B-1) mit Kupfer, Kobalt oder Mangan und deren Gemischen ausgewählt ist. In einer speziellen Ausführungsform setzt man wenigstens ein unter CuCl, CuI, $CuCl_2$, $CoCl_2$ und deren Mischungen ausgewähltes Salz ein. In einer anderen speziellen Ausführungsform des erfindungsgemäßen Verfahrens setzt man als Übergangsmetall wenigstens ein Acetylacetonat, Oxalat, Naphthenat oder $C_2$-$C_{18}$-Alkanoat des Kobalts oder des Mangans oder einen Komplex des Kobalts oder des Mangans mit N,N-Salicyliminoethan (Ligand B1) ein, z.B. Kobaltbisacetylacetonat, Kobalt(II)-2-ethylhexanoat, Kobalt(III)trisacetylacetonat, Kobalt(II)oxalat, Kobalt(II)acetat, Kobalt(II)naphthenat, Kobalt(II)salicyliminoethan, Chlorokobalt(III)salicyliminoethan, Mangan(II)salicyliminoethan, Chloromangan(III)salicyliminoethan, Mangantrisacetylacetonat oder ein Manganbisacetylacetonat-Alkanoat. In einer weiteren speziellen Ausführungsform des erfindungsgemäßen Verfahrens setzt man als Übergangsmetall wenigstens ein Acetylacetonat, Naphthenat, $C_2$-$C_{18}$-Alkanoat oder einen N,N-Salicyliminoethan-Komplex des Kupfers ein, z.B. Kupfer(II)acetat.

In einer ganz speziellen Ausführungsform des erfindungsgemäßen Verfahrens setzt man als Übergangsmetall wenigstens ein Salz des Kupfers, des Mangans oder des Kobalts, insbesondere wenigstens ein Halogenid des Kupfers oder des Kobalts, speziell CuCl, $CoCl_2$ oder CuI, oder wenigstens ein Acetylacetonat, $C_2$-$C_{18}$-Alkanoat, Oxalat, Naphthenat oder N,N-Salicyliminoethan-Komplex des Kupfers, des Kobalts oder des Mangans ein, z.B. Kobaltbisacetylacetonat, Kobalt(II)-2-ethylhexanoat, Kupfer(II)acetat, Kobalt(III)trisacetylacetonat, Kobalt(II)oxalat, Kobalt(II)acetat, Kobalt(II)naphthenat, Kobalt(II)salicyliminoethan, Chlorokobalt(III)salicyliminoethan, Mangan(II)salicyliminoethan, Chloromangan(III)-salicyliminoethan, Mangantrisacetylacetonat oder Manganbisacetylacetonat-Alkanoat, und führt die Umsetzung in Gegenwart eines oder mehrerer der im Zusammenhang mit den Übergangsmetallkomplexverbindungen genannten Liganden, insbesondere in Gegenwart wenigstens eines Liganden, der wenigstens ein Stickstoffatom enthält, und speziell wenigstens eines Liganden aus der Gruppe Pyridin, Bipyridin-Verbindungen, Phenanthrolin-Verbindungen, Oxazolverbindungen und Liganden der allgemeinen Formeln A, B und C, ganz speziell aus der Gruppe der Liganden, die eine Oxazol-, 2,2-Bipyridin- und 1,10-Phenanthrolingruppe aufweisen, und speziell in Gegenwart von Pyridin oder in Gegenwart von Phenanthrolin, Bipyridin oder Oxazol durch.

In einer weiteren ganz speziellen Ausführungsform setzt man wenigstens ein Acetylacetonat, Naphthenat, Oxalat oder $C_2$-$C_{18}$-Alkanoat des Kupfers, des Kobalts oder des Mangans oder einen N,N-Salicyliminoethankomplex des Kupfers, des Kobalts oder des Mangans ein, z.B. Kobaltbisacetylacetonat, Kobalt(II)-2-ethylhexanoat, Kobalttrisacetylacetonat, Kupfer(II)acetat, Kobalt(II)oxalat, Kobalt(II)acetat, Kobalt(II)-naphthenat, Kobalt(II)salicyliminoethan, Chlorokobalt(III)salicyliminoethan, Mangan(II)salicyliminoethan, Chloromangan(III)salicyliminoethan, Mangantrisacetylacetonat oder ein Manganbisacetylacetonat-Alkanoat, und führt die Umsetzung in einem organischen Lösungsmittel oder Lösungsmittelgemisch durch, das unter $C_1$-$C_4$-Alkylestern der Essigsäure, z.B. Ethylacetat, Acetonitril, Dimethylformamid, N-Methylpyrrolidon oder Toluol, deren Gemischen und Gemischen eines oder mehrerer dieser Lösungsmittel mit Pyridin ausgewählt ist. In diesen Gemischen beträgt die Menge an Pyridin typischerweise 1 bis 10 Mol pro mol Übergangsmetall.

In einer weiteren ganz speziellen Ausführungsform setzt man wenigstens eine Übergangsmetallverbindung ein, die unter Acetylacetonaten, Naphthenaten, $C_2$-$C_{18}$-Alkanoaten des Kupfers, des Kobalts oder des Mangans und N,N-Salicyliminoethan-komplexen des Kupfers, des Kobalts oder des Mangans ausgewählt ist, z.B. Kobaltbisacetylacetonat, Kobalttrisacetylacetonat, Kupfer(II)acetat, Kobalt(II)oxalat, Kobalt(II)acetat, Kobalt(II)naphthenat, Kobalt(II)-2-ethylhexanoat, Kobalt(II)salicyliminoethan, Chlorokobalt(III)salicyliminoethan, Mangan(II)salicyliminoethan, Chloromangan(III)salicyliminoethan, Mangantrisacetylacetonat oder ein Manganbisacetylacetonat-Alkanoat, und führt die Umsetzung in einem organischen Lösungsmittel oder Lösungsmittelgemisch durch, das unter $C_1$-$C_4$-Alkylestern der Essigsäure, z.B. Ethylacetat, Acetonitril, Dimethylformamid, N-Methylpyrrolidon oder Toluol ausgewählt ist, und in Gegenwart wenigstens eines weiteren Liganden, insbesondere in Gegenwart wenigstens eines Liganden, der wenigstens ein Stickstoffatom enthält, und speziell wenigstens eines Liganden aus der Gruppe Bipyridin-Verbindungen, Phenanthrolin-Verbindungen und Oxazolverbindungen und ganz speziell in Gegenwart eines Phenanthrolin-Liganden. In dieser ganz

speziellen Ausführungsform liegt das Molverhältnis von Metall zu Ligand vorzugsweise im Bereich von 0,5 : 1 bis 2:1.

In einer weiteren ganz speziellen Ausführungsform setzt man wenigstens eine Übergangsmetallverbindung ein, die unter Acetylacetonaten, Naphthenaten, $C_2$-$C_{18}$-Alkanoaten des Kupfers, des Kobalts oder des Mangans und N,N-Salicyliminoethan-komplexen des Kupfers, des Kobalts oder des Mangans ausgewählt ist, z.B. Kobaltbisacetylacetonat Kobalttrisacetylacetonat, Kupfer(II)acetat, Kobalt(II)oxalat, Kobalt(II)acetat, Kobalt(II)-2-ethylhexanoat, Kobalt(II)naphthenat, Kobalt(II)salicyliminoethan, Chlorokobalt(III)salicyliminoethan, Mangan(II)-salicyliminoethan, Chloromangan(III)salicyliminoethan, Mangantrisacetylacetonat oder ein Manganbisacetylacetonat-Alkanoat, und führt die Umsetzung in Abwesenheit eines organischen Lösungsmittels durch. In dieser Ausführungsform kann die Umsetzung in Abwesenheit weiterer Liganden und insbesondere in Gegenwart wenigstens eines weiteren Liganden, insbesondere in Gegenwart wenigstens eines Liganden, der wenigstens ein Stickstoffatom enthält, und speziell in Gegenwart wenigstens eines Liganden aus der Gruppe Bipyridin-Verbindungen, Phenanthrolin-Verbindungen und Oxazolverbindungen und ganz speziell in Gegenwart eines Phenanthrolin-Liganden durchgeführt werden. In dieser ganz speziellen Ausführungsform liegt das Molverhältnis von Metall zu Ligand, sofern eingesetzt, vorzugsweise im Bereich von 0,5 : 1 bis 2:1.

In einer weiteren ganz speziellen Ausführungsform setzt man wenigstens eine Übergangsmetallverbindung ein, die unter Verbindungen des Kobalts, insbesondere unter Acetylacetonaten, Naphthenaten und $C_2$-$C_{18}$-Alkanoaten des Kobalts und N,N-Salicyliminoethankomplexen des Kobalts ausgewählt ist, z.B. Kobaltbisacetylacetonat, Kobalttrisacetylacetonat, Kobalt(II)-2-ethylhexanoat, Kobalt(II)acetat, Kobalt(II)oxalat, Kobalt(II)naphthenat, Kobalt(II)salicyliminoethan, Chlorokobalt(III)salicyliminoethan, und führt die Umsetzung in einem organischen Lösungsmittel oder Lösungsmittelgemisch durch, das unter $C_1$-$C_4$-Alkylestern der Essigsäure, z.B. Ethylacetat, Acetonitril, Dimethylformamid, N-Methylpyrrolidon oder Toluol, und in Gegenwart wenigstens eines weiteren Liganden ausgewählt ist, insbesondere in Gegenwart wenigstens eines Liganden aus der Gruppe der Phenanthrolin-Verbindungen. In dieser ganz speziellen Ausführungsform liegt das Molverhältnis von Metall zu Phenanthrolin-Verbindung vorzugsweise im Bereich von 0,5 : 1 bis 2:1.

In einer weiteren ganz speziellen Ausführungsform setzt man wenigstens eine Übergangsmetallverbindung ein, die unter Acetylacetonaten, Naphthenaten, $C_2$-$C_{18}$-Alkanoaten des Kobalts und N,N-Salicyliminoethankomplexen des Kobalts ausgewählt ist, z.B. Kobaltbisacetylacetonat, Kobalttrisacetylacetonat, Kobalt(II)oxalat, Kobalt(II)-2-ethylhexanoat, Kobalt(II)acetat, Kobalt(II)naphthenat, Kobalt(II)salicyliminoethan, Chlorokobalt(III)salicyliminoethan, und führt die Umsetzung in Gegenwart wenigstens eines weiteren Liganden, insbesondere in Gegenwart wenigstens eines Liganden aus der Gruppe der Phenanthrolin-Verbindungen und in Abwesenheit von Lösungsmitteln durch. In dieser ganz speziellen Ausführungsform liegt das Molverhältnis von Metall zu Phenanthrolin-Verbindung vorzugsweise im Bereich von 0,5 : 1 bis 2:1.

Typischerweise setzt man das Übergangsmetall in einer Menge von $5x10^{-6}$ bis 0,5 mol, insbesondere in einer Menge von $5x10^{-5}$ bis 0,3 mol und speziell in einer Menge von $5x10^{-4}$ bis 0,3 mol pro mol der Verbindung der Formel II ein. Im Falle von Kupfer, Kobalt oder Mangan setzt man das Übergangsmetall in der Regel in einer Menge von $5x10^{-5}$ bis 0,5 mol, insbesondere in einer Menge von $10^{-4}$ bis 0,3 mol pro mol der Verbindung der Formel II ein.

Typischerweise erfolgt die Umsetzung der Verbindung der Formel II mit dem Oxidationsmittel in einem Lösungs- bzw. Verdünnungsmittel. Als Lösungs-/Verdünnungsmittel kommen grundsätzlich alle unter Reaktionsbedingungen inerten organischen Lösungsmittel sowie Wasser in Betracht. Geeignete, inerte organische Lösungsmittel sind halogenierte Lösungsmittel wie Dichlormethan (DCM), Chloroform, Dichlorethan (DCE) und Chlorbenzol, weiterhin polare aprotische Lösungsmittel wie Pyridin, Acetonitril, N-Methylpyrrolidon, Dimethylformamid oder die $C_1$-$C_4$-Alkylester der Essigsäure sowie aliphatische und cycloaliphatische Kohlenwasserstoffe wie Hexan, Heptan, Octan und deren Isomerengemische, Cylohexan, Methylcyclohexan und Cycloheptan, sowie aromatische Kohlenwasserstoffe wie Benzol, Toluol, Xylole, weiterhin Gemische der vorgenannten organischen Lösungsmittel und Gemische davon mit Wasser. Als Lösungs-/Verdünnungsmittel kommen auch ionische Flüssigkeiten wie (Di)alkyl-imidazoliumsalze, z.B. deren Chloride, Bromide, Trifluoracetate, Sulfonate, Sulfat, Hydrogensulfate, Metosulfate, Carbonate, Hydrogencarbonate und Triflate in Frage. Auch eine Durchführung im 2-Phasensystem Wasser und organisches Lösungsmittel ist vorteilhaft. Hierbei können auch Phasentransferkatalysatoren wie z. B. Tetraalkylammoniumsalze oder Trialkylbenzylammoniumsalze, z.B. Tetra-$C_1$-$C_4$-alkylammoniumhalogenide, Tri-$C_1$-$C_4$-alkylbenzylammoniumhalogenide oder Tri-$C_1$-$C_4$-alkyl-$C_6$-$C_{20}$-alkylammoniumhalogenide zugesetzt werden.

In einer bevorzugten Ausführungsform der Erfindung umfasst das Lösungs- bzw. Verdünnungsmittel wenigstens ein unter Pyridin, Acetonitril und Essigester ausgewähltes organisches Lösungsmittel. In einer weiteren bevorzugten Ausführungsform der Erfindung umfasst das Lösungs- bzw. Verdünnungsmittel wenigstens ein unter Methylpyrrolidon und Dimethylformamid ausgewähltes organisches Lösungsmittel.

Sofern die Umsetzung der Verbindung II mit dem Oxidationsmittel in einem Lösungs- bzw. Verdünnungsmittel durchgeführt wird, wird man die Konzentration an Verbindung II typischerweise im Bereich von 5 bis 70 Gew.-%, häufig 5 bis 60 Gew.-% und speziell 5 bis 50 Gew.-%, bezogen auf das Gesamtgewicht der Reaktionsmischung, wählen.

Die zur Umsetzung der Verbindung der Formel II mit dem Oxidationsmittel erforderlichen Reaktionstemperaturen können vom Fachmann durch Routinemaßnahmen ermittelt werden und liegen typischerweise im Bereich von 0

bis 100°C, häufig im Bereich von 15 bis 100°C, vorzugsweise im Bereich von 20 bis 100°C und insbesondere im Bereich von 30 bis 95°C. Der Reaktionsdruck hat auf die Umsetzung keine oder nur geringe Auswirkung und liegt daher in der Regel im Bereich von 700 bis 1500 mbar, wobei auch geringere oder höhere Drucke möglich sind. Die Umsetzung kann in einer inerten Atmosphäre durchgeführt werden, z.B. unter Inertgas wie Stickstoff oder Argon. Die zur Umsetzung der Verbindung der Formel II mit dem Oxidationsmittel erforderlichen Reaktionszeiten können vom Fachmann durch Routinemaßnahmen ermittelt werden und liegen typischerweise im Bereich von 10 min. bis 48 h, insbesondere im Bereich von 30 min. bis 24 h.

Zur Umsetzung wird man typischerweise die Verbindung der Formel II mit dem Oxidationsmittel und dem wenigstens einen Übergangsmetall, vorzugsweise der wenigstens einen Übergangsmetallverbindung in einem geeigneten Reaktionsgefäß in Kontakt bringen. Hierzu kann man beispielsweise die Verbindung der Formel II in einem geeigneten Reaktionsgefäß vorlegen und hierzu das Oxidationsmittel und das wenigstens eine Übergangsmetall zugeben. Man kann auch die Verbindung der Formel II zusammen mit dem wenigstens einen Übergangsmetall in einem geeigneten Reaktionsgefäß vorlegen und hierzu das Oxidationsmittel zugeben. Man kann auch die Verbindung der Formel II zusammen mit dem wenigstens einen Übergangsmetall und dem Oxidationsmittel in einem geeigneten Reaktionsgefäß vorlegen und dann auf Reaktionstemperatur erwärmen.

Das Oxidationsmittel kann in reiner Form oder als Lösung in Wasser oder einem inerten organischen Lösungsmittel oder Gemischen inerter organischer Lösungsmittel oder Gemischen von Wasser und inerten organischen Lösungsmitteln eingesetzt werden. Hier sind insbesondere $C_6$-$C_{20}$-Alkane, Acetonitril, $C_1$-$C_4$-Alkylester der Essigsäure wie Essigsäureethylester oder Pyridin und deren Gemische zu nennen. Die Konzentration an Peroxid liegt dann vorzugsweise im Bereich von 10 bis 90 Gew.-%, insbesondere im Bereich von 30 bis 70 Gew.-%. Gemäß einer bevorzugten Ausführungsform wird als Oxidationsmittel ein Alkylperoxid in Form einer wässrigen Lösung eingesetzt. Die Konzentration an Alkylperoxid in der wässrigen Lösung liegt dann vorzugsweise im Bereich von 10 bis 90 Gew.-%, insbesondere im Bereich von 30 bis 70 Gew.-%. Das Alkylperoxid kann zusätzlich mit Anteilen eines oder mehrerer der für die Reaktion geeigneten und zuvor genannten, organischen Lösungsmittel verdünnt werden. Bevorzugt werden 0,1 bis 10 Mol, besonders bevorzugt 1 bis 5 Mol Lösungsmittel pro Mol Verbindung der Formel II eingesetzt werden. Es kann hier auch ein zweites Lösungsmittel eingesetzt werden. Das Alkylperoxid kann aber auch in einem organischen Lösungsmittel gelöst eingesetzt werden, insbesondere in aliphatischen $C_6$-$C_{12}$-Kohlenwasserstoffen (verzweigt oder unverzweigt). Bevorzugte $C_6$-$C_{12}$-Kohlenwasserstoffe sind Decan oder Undecan und deren Gemische. Das Alkylperoxid kann aber auch in Form einer Lösung in Dialkylperoxid eingesetzt werden. Als Beispiel hierfür ist Luperox® als kommerziell erhältliche Form zu nennen.

Das Oxidationsmittel kann in einer Portion vorgelegt oder zu Beginn der Umsetzung in einer Portion zugegeben werden. Das Oxidationsmittel kann auch teilweise oder vollständig im Verlauf der Umsetzung zugegeben werden.

Selbstverständlich kann man das wenigstens eine Übergangsmetall zu verschiedenen Zeitpunkten zugeben, z.B. eine Teilmenge zu Beginn der Umsetzung zugeben oder im Reaktionsansatz vorlegen und im Verlauf der Reaktion die verbleibende Menge an Übergangsmetall in einer oder mehreren Portionen zugeben. Das im Verlauf der Umsetzung zugegebene wenigstens eine weitere Übergangsmetall kann von dem zu Beginn der Umsetzung zugegebenen oder im Reaktionsansatz vorgelegten Übergangsmetall gleich oder verschieden sein.

In einer speziellen Ausführungsform der Erfindung führt man die Umsetzung der Verbindung der Formel II so durch, dass man zunächst die unten beschriebene Verbindung der Formel III herstellt, diese ggf. isoliert und anschließend die Verbindung der Formel III zu der Verbindung der Formel I zersetzt.

Die Herstellung der Verbindung der Formel I aus der Verbindung II über die Zwischenstufe III kann beispielsweise dadurch erfolgen, dass man zunächst nur eine Teilmenge des wenigstens einen Übergangsmetalls zu Beginn der Umsetzung in das Reaktionsgefäß gibt, wodurch primär die Verbindung der Formel III gebildet wird. Anschließend wird die Verbindung der Formel III zersetzt, beispielsweise durch Erhitzen, durch Zugabe von weiterem Übergansmetall, durch Zugabe einer Base, insbesondere einer Stickstoffbase, speziell unter Zugabe eines sekundären oder tertiären Amins oder durch Kombination einer oder mehrerer dieser Maßnahmen.

Die Herstellung der Verbindung der Formel I über die Zwischenstufe III kann beispielsweise auch dadurch erfolgen, dass man die Verbindung der Formel II mit dem Oxidationsmittel in Gegenwart des Übergangsmetalls und gegebenenfalls eines oder mehrerer der zuvor beschriebenen Liganden bei niedrigen Temperaturen umsetzt, wobei primär die Verbindung der Formel III gebildet wird, und diese anschließend zur Verbindung der Formel I zersetzt, beispielsweise durch Erhitzen, durch Zugabe einer Base, insbesondere einer Stickstoffbase, speziell unter Zugabe eines sekundären oder tertiären Amins oder durch Kombination einer oder mehrerer dieser Maßnahmen. Bevorzugte Temperaturen für die Umsetzung der Verbindung II zur Verbindung III liegen im Bereich von 0 bis 100 °C, insbesondere im Bereich von 10 bis 60 °C.

Gemäß einer Ausgestaltung erfolgt die Zersetzung der Verbindung III zur Verbindung I thermisch. Hierzu wird die Verbindung der Formel III oder das Reaktionsgemisch aus der Umsetzung der Verbindung II mit dem Oxidationsmittel auf Temperaturen von in der Regel wenigstens 40 °C, häufig wenigstens 50 °C und insbesondere wenigstens 60 °C, z.B. 40 bis 100 °C, insbesondere 50 bis 95 °C und speziell 60 bis 90 °C erhitzt. Gegebenenfalls kann man zuvor

leichtsiedende Bestandteile aus dem Reaktionsgemisch destillativ entfernen. Die thermische Zersetzung wird dabei vorzugsweise so lange durchgeführt, bis wenigstens 90 %, insbesondere die Gesamtmenge der Verbindung der Formel III umgesetzt ist. Die hierfür erforderliche Zeit hängt naturgemäß von der Art des Reaktionsgemischs und der Temperatur ab und liegt typischerweise im Bereich von 2 bis 24 h.

Gemäß einer anderen Ausgestaltung erfolgt die Zersetzung der Verbindung III zur Verbindung I durch Behandlung mit Base, wobei das Reaktionsgemisch zuvor bzgl. der Verbindung III angereichert werden kann, z.B. durch destillatives Entfernen niedrig siedender Bestandteile. Zur Behandlung der Verbindung III mit der Base wird die Verbindung der Formel III oder das Reaktionsgemisch aus der Umsetzung der Verbindung II mit der Base versetzt, wobei man die Base zu der Verbindung der Formel III oder zu dem Reaktionsgemisch aus der Umsetzung der Verbindung II geben kann oder die Verbindung der Formel III oder das Reaktionsgemisch aus der Umsetzung der Verbindung II mit dem Oxidationsmittel zu der Base gibt. Die Basenmenge liegt typischerweise im Bereich von 1 bis 10 Mol, insbesondere 1 bis 5 Mol und speziell 1 bis 3 Mol pro Mol Verbindung der Formel III. Geeignete Basen sind Stickstoffbasen, insbesondere sekundäre oder tertiäre Mono- oder Diamine, insbesondere $C_1$-$C_{10}$-Trialkylamine, Tetra-$C_1$-$C_4$-alkyl-diaminoalkane und gesättigte 5 bis 7-gliedrige Stickstoffhetereocyclen, die ggf. am Stickstoffatom eine $C_1$-$C_{10}$-Alkylgruppe tragen sowie Oxobasen, insbesondere Alkalimetallcarbonate,-hydorgencarbonate, -hydroxide und $C_1$-$C_{10}$-Alkoholate, insbesondere die entsprechenden Natrium-, Kalium- und Lithiumverbindungen, Erdalkalimetallhydroxide und $C_1$-$C_{10}$-Alkoholate, insbesondere die entsprechenden Calcium- und Magnesiumverbindungen, sowie Ammoniumcarbonat, Ammoniumhydrogencarbonate und Ammoniumhydroxid. Beispiele für Stickstoffbasen sind insbesondere Morpholin, Piperidin, N-Methylpiperidin, Triethylamin, Diisopropylethylamin und Tripropylamin sowie N,N,N',N'-Tetramethyl-1,2-diaminoethan. Beispiele für Oxobasen sind vor allem Natriummethanolat, Kaliummethanolat, Kalium-tert.-Butanolat, Lithiumhydroxid, Calciumhydroxid und Magnesiumhydroxid. Die Reaktionsdauer für die durch Basen initiierte Zersetzung von III liegt typischerweise im Bereich von 1 h bis 24 h. Die Reaktionstemperatur für die durch Basen initiierte Zersetzung von III liegt typischerweise im Bereich von 25°C bis 100°C.

In einer weiteren speziellen Ausführungsform gibt man eine Teilmenge des wenigstens einen Übergangsmetalls zu Beginn der Umsetzung in das Reaktionsgefäß oder legt sie darin vor. Hierbei bildet sich zunächst das Zwischenprodukt der Formel III (s.u.), das dann bei Zugabe von weiterem Übergangsmetall, das identisch mit dem zuerst zugegebenen Übergangsmetall oder davon verschieden sein kann, in die Verbindung I umgewandelt wird. Beispielsweise kann man die Oxidation von II in die Verbindung I so durchführen, dass man zunächst die Verbindung II mit dem Oxidationsmittel in Gegenwart des wenigstens einen Übergangsmetalls in die im Folgenden beschriebene Verbindung III überführt und diese durch Zugabe wenigstens eines der hier als bevorzugt angegebenen Übergangsmetalle, bei dem es sich vorzugsweise um Kupfer, Kobalt oder Mangan und speziell um Kupfer handelt, vorzugsweise in Form einer Übergangsmetallverbindung, insbesondere in Form eines Komplexes oder Salzes, insbesondere Kupfer in Form eines Salzes, wie CuCI oder Cul, zur Verbindung I weiter umsetzt. Speziell wird das weitere Übergangsmetall, bei dem es sich vorzugsweise um Kupfer handelt, in Form einer Lösung einer geeigneten Übergangsmetallverbindung, insbesondere in Form einer Lösung eines geeigneten Übergangsmetallsalzes wie CuCI oder Cul, in einem organischen Lösungsmittel oder Lösungsmittelgemisch, das Pyridin enthält, zugegeben. Die Menge an weiterem Übergangsmetall liegt bei etwa $5x10^{-4}$ bis 0,05 mol, insbesondere im Bereich von $10^{-3}$ bis 0,03 mol pro mol Verbindung II.

Vorzugsweise sorgt man während der Umsetzung für eine Durchmischung des Reaktionsansatzes, z.B. durch Rühren oder durch Umpumpen.

Die Umsetzung liefert die gewünschte Verbindung der Formel I in guten Ausbeuten. Hierbei wird primär die Verbindung der Formel III gebildet, die sich dann in die Verbindung I umwandelt, vorzugsweise unter Erhitzen und/oder Zugabe von weiterem Übergansmetall und/oder durch Zugabe einer Base, insbesondere einer Stickstoffbase, speziell unter Zugabe eines sekundären oder tertiären Amins oder einer Oxobase.

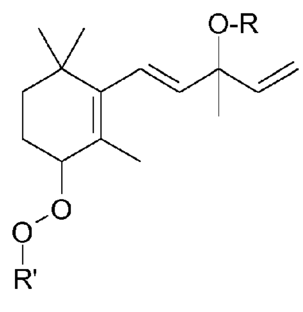

(III)

In Formel III hat R die für Formel I angegebenen Bedeutungen, insbesondere die dort als bevorzugt angegebenen Bedeutungen und steht speziell für Wasserstoff. R' steht für tertiäres $C_4$-$C_8$-Alkyl wie tert-Butyl oder 1,1-Dimethylpropyl (= tert. Amyl) oder für tertiäres Phenyl-$C_3$-$C_8$-alkyl, wobei der Phenylrest unsubstituiert ist oder 1, 2 oder 3

$C_1$-$C_4$-Alkylgruppen tragen kann, z.B. für 2-Phenyl-2-propyl (Cumyl).

Die Verbindung III kann aus dem primären Reaktionsgemisch abgetrennt werden, beispielsweise auf chromatographischem Wege, z. B. mittels Säulenchromatographie an Kieselgel mit Essigsäureethylester/$C_6$-$C_8$-Alkan- oder Tetrahydrofuran/$C_6$-$C_8$-Alkan-Gemischen. Üblicherweise wird man die Verbindung jedoch zur Verbindung I weiter umsetzen, z. B. durch Erhitzen und/oder weitere Zugabe von Übergangsmetall und/oder durch Zugabe einer Base, insbesondere einer Stickstoffbase, speziell unter Zugabe eines sekundären oder tertiären Amins oder einer Oxobase, oder es findet eine spontane Umwandlung der Verbindung III in die Verbindung der Formel I statt, z. B. bei längerer Reaktionsdauer und/oder höheren Reaktionstemperaturen.

Die Verbindungen der Formel III sind neu und ebenfalls Gegenstand der vorliegenden Erfindung.

Die Aufarbeitung des bei der Oxidation von II anfallenden Reaktionsgemisches erfolgt in üblicher Weise. Gegebenenfalls wird man zur Zerstörung eines Überschusses des Oxidationsmittels dieses vor einer Isolierung der Verbindung I zersetzen, z.B. durch Zusatz eines Reduktionsmittels wie beispielsweise Natriumsulfit, Natriumhydrogensulfit, Eisen(II)sulfat oder Natriumthiosulfat. Die weitere Aufarbeitung kann wässrig extraktiv oder in sonstiger Weise erfolgen. Gegebenenfalls führt man im Anschluss eine Aufreinigung der so erhaltenen Verbindung der Formel I durch, z.B. durch Destillation oder Chromatographie oder Kombinationen dieser Methoden.

Ein weiterer Gegenstand der Erfindung ist die Herstellung von Verbindungen der Formel IV, welche in einem ersten Schritt die Herstellung einer Verbindung der allgemeinen Formel I nach dem erfindungsgemäßen Verfahren und in einem zweiten Schritt die Umwandlung der Verbindung der Formel I in die Verbindung der Formel IV in an sich bekannter Weise umfasst. Beispielsweise kann die Umwandlung der Verbindung der Formel I, worin X für OH steht und R für Wasserstoff oder eine OH-Schutzgruppe steht, vorzugsweise eine durch Säure abspaltbare OH-Schutzgruppe, z.B. eine Gruppe der Formel $SiR^aR^bR^c$ oder eine Acetalschutzgruppe, nach den in EP 101597 und EP 490326 beschriebenen Methode erfolgen.

Beispielsweise kann man in Analogie zu der in EP 101597 beschriebenen Methode die Verbindung der Formel I in einen Silylenolether der Formel V umwandeln.

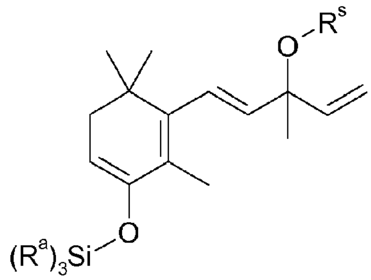

(V)

In Formel V können die Reste $R^a$ in der Gruppe $Si(R^a)_3$ gleich oder verschieden sein und stehen unabhängig voneinander für $C_1$-$C_4$-Alkyl und speziell für Methyl oder Ethyl. $R^s$ steht für eine OH-Schutzgruppe, vorzugsweise eine durch Säure bzw. Säurebehandlung abspaltbare OH-Schutzgruppe, z.B. eine Gruppe der Formel $SiR^aR^bR^c$ oder eine Acetalschutzgruppe und speziell für eine Gruppe $Si(R^a)_3$.

Die Umwandlung der Verbindung I zur Verbindung der Formel V gelingt beispielsweise durch Umsetzung einer Verbindung der Formel I mit einem Trialkylsilylhalogenid oder Pseudohalogenid der Formel $HalSi(R^a)_3$, wobei Hal für Halogen und insbesondere für Chlor oder für ein Pseudohalogenid, insbesondere für Mesilat oder Triflat steht. Besonders bevorzugt gelingt die Umwandlung zur Verbindung der Formel V, wenn man eine Verbindung der Formel $HalSi(R^a)_3$ einsetzt, worin Hal für Mesilat oder Triflat steht und worin $R^a$ vorzugsweise für Methyl oder Ethyl steht. Die Verbindung $HalSi(R^a)_3$ wird typischerweise in der stöchiometrisch benötigten Menge oder im Überschuss eingesetzt, im Falle der Verbindung I mit R = H vorzugsweise im Bereich 2 bis 10 Äquivalenten, insbesondere in einer Menge von 5 bis 9 Äquivalenten, bezogen auf die Stoffmenge an Verbindung der Formel I mit R = H. Sofern in der eingesetzten Verbindung der Formel I die Variable R für Wasserstoff steht, erhält man einen Silylenolether der Formel V, worin $R^s$ für eine Gruppe $Si(R^a)_3$ steht.

Die Umsetzung der Verbindung I zur Verbindung der Formel V erfolgt in der Regel in Gegenwart eines tertiären Amins, beispielsweise eines Tri-$C_1$-$C_4$-alkylamins oder Di-$C_1$-$C_4$-alkyl-$C_5$-$C_8$-cycloalkylamins wie Triethylamin, Diisopropylethylamin, Tri-n-propylamin, Diethylcyclohexylamin, einer Amidinbase wie 1,5-Diazabicyclo[4.3.0]non-5-en (DBN) oder 1,8-Diazabicyclo[5.4.0]undec-7-en (DBU) oder eines polycyclischen Amins wie 1,4-Diazabicyclo[2.2.2]octan (DABCO). Besonders bevorzugt ist Triethylamin. Die Menge an tertiärem Amin liegt typischerweise im Bereich von 2 bis 15 Äquivalenten, insbesondere im Bereich von 6 bis 11 Äquivalenten, bezogen auf die Stoffmenge an Verbindung der Formel I mit R = H.

Die Umsetzung der Verbindung I zur Verbindung der Formel V erfolgt in der Regel in einem organischen Lösungsmittel oder Lösungsmittelgemisch. Geeignete Lösungsmittel sind insbesondere aprotische Lösungsmittel, wie

beispielsweise N,N-Dimethylformamid, N,N-Dimethylacetamid, Tetrahydrofuran, Methyltetrahydrofuran, Dichlormethan, Dichlorethan, Acetonitril, Propylencarbonat und Toluol sowie deren Gemische. Üblicherweise setzt man das Lösungsmittel in einer Menge im Bereich von 0,5 bis 10 g, insbesondere 1 bis 3 g pro g Verbindung der Formel I ein.

Gegebenenfalls kann die Umsetzung der Verbindung I zur Verbindung der Formel V in Gegenwart eines Phasentransferkatalysators, beispielsweise eines Tetra-$C_1$-$C_{12}$-alkylammoniumsalzes, insbesondere in Gegenwart eines Tetra-$C_1$-$C_{12}$-alkylammoniumhydroxids, -chlorids, -bromids, -iodids, -mesylats oder -triflats wie Tetrabutylammoniumhydroxid, Tetrabutylammoniumchlorid, Tetrabutylammoniumbromid, Tetrabutylammoniumiodid, Tetrabutylammoniummesylat, Tetrabutylammoniumtriflat und/oder in Gegenwart eines Di-$C_1$-$C_4$-alkylaminopyridins wie 4-Dimethylaminopyridin, durchgeführt werden.

Wegen weiterer Details wird hiermit auf die EP 101597, insbesondere auf die Seiten 5 und 6 und die Beispiele 6 und 8 verwiesen.

Anschließend wird der Silylenolether der Formel V mit einer Peroxocarbonsäure, beispielsweise Peressigsäure, Perbenzoesäure oder Monoperphthalsäure, umgesetzt, wobei man die Verbindung der Formel VI erhält:

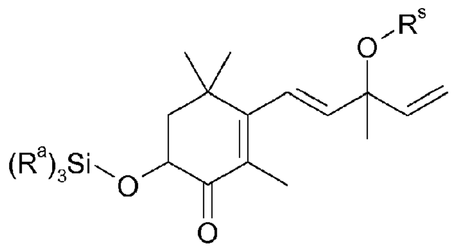

(VI)

worin die Reste $R^a$ in den Gruppen $Si(R^a)_3$ gleich oder verschieden sein können und unabhängig voneinander für $C_1$-$C_4$-Alkyl stehen und worin $R^s$ die zuvor für den Silylenolether der Formel V genannten Bedeutungen aufweist und insbesondere für eine Gruppe der Formel $Si(R^a)_3$ steht. Hierbei fällt die Verbindung der Formel (VI) häufig im Gemisch mit der Verbindung der Formel VIIa, und/oder, wenn $R^s$ in Formel V für eine Gruppe der Formel $Si(R^a)_3$ steht, im Gemisch mit dem freien Alkohol der Formel VII an.

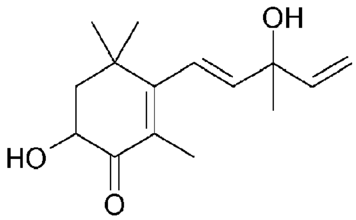

(VII)

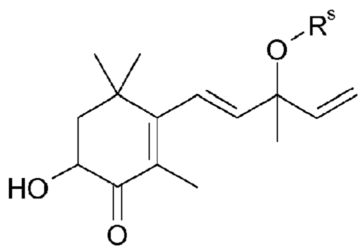

(VIIa)

In Formel VIIa , steht $R^s$ für eine OH-Schutzgruppe, insbesondere eine durch Säure abspaltbare OH-Schutzgruppe, speziell für eine Acetalschutzgruppe oder ganz speziell für eine Gruppe $Si(R^a)_3$, wobei $R^a$ gleich oder verschieden sind und für $C_1$-$C_4$-Alkyl stehen.

Abspaltung der Schutzgruppe $R^s$ aus der Verbindung VI bzw. VIIa und gegebenenfalls der Gruppe $Si(R^a)_3$ aus der Verbindung VI, z.B. durch Hydrolyse der Verbindung der Formel VI, beispielsweise unter Einsatz von Trialkylammoniumhydrofluorid, wenn $R^s$ für $Si(R^a)_3$ steht, liefert anschließend die Verbindung der Formel VII.

Wegen weiterer Details wird hiermit auf die EP 101597, insbesondere auf die Seiten 5 und 6 und die Beispiele 7 und 9 verwiesen.

Sukzessive Umsetzung der Verbindung der Formel VII mit einem Halogenierungsmittel, vorzugsweise einem Halogenwasserstoff HZ, insbesondere Bromwasserstoff, und anschließend mit Triphenylphosphan, liefert in guten Ausbeuten die Verbindung der Formel IV, worin X für OH steht. Vorzugsweise wird man vor der Umsetzung mit Triphenylphosphan überschüssigen Halogenwasserstoff HZ entfernen. Insbesondere hat es sich als vorteilhaft erwiesen, vor der Umsetzung mit Triphenylphosphan etwaige Spuren an Halogenwasserstoff durch Zusatz von Alkylenoxid, wie Butylenoxid, zu entfernen (vgl. Helv. Chim. Acta 64, 2444, 1981).

Die Reinigung des Phosphonium-Salzes kann durch Kristallisation und Wäsche bzw. Umkristallisation erfolgen. Zur Kristallisation bzw. Umkristallisation werden insbesondere Dichlormethan, Dichlorethan, Chlorbenzol, Toluol, Heptan, Cyclohexan, Methylcyclohexan, THF, iso-Propanol, iso-Butanol, Essigsäure $C_1$-$C_4$-Alkylester, Acetonitril oder Aceton einzeln oder deren Gemische eingesetzt.

Alternativ kann man zur Herstellung der Verbindung IV zunächst eine Verbindung der Formel I, worin R für eine OH-Schutzgruppe, insbesondere eine durch Säure abspaltbare OH-Schutzgruppe $R^s$ und insbesondere für eine Gruppe $Si(R^a)_3$ steht, wobei $R^a$ gleich oder verschieden sind und für $C_1$-$C_4$-Alkyl stehen, in ihr Enolat umwandeln. Die Umwandlung in das Enolat gelingt durch Umsetzung von I mit einer starken Base, beispielsweise einem Alkalimetallamid wie Lithiumdiisopropylamid, Lithium(hexamethyldisilazan), Natrium(hexamethyldisilazan) oder Kalium(hexamethyldisilazan) in Analogie zu der in EP 490326 beschriebenen Methode. Das so erhaltene Enolat wird anschließend mit einem N-Arylsulfonyloxaziridin, z.B. N-Phenylsulfonyl-3-phenyloxaziridin, umgesetzt, wobei man die zuvor definierte Verbindung der Formel VIIa erhält.

Wegen weiterer Details wird hiermit auf die EP 490326, insbesondere die darin enthaltenen Beispiele verwiesen.

Abspaltung der Schutzgruppe $R^s$ aus der Verbindung der Formel VIIa, z.B. durch saure Hydrolyse, Umsetzung des dabei erhaltenen Produkts mit einem Halogenierungsmittel, beispielsweise einem Halogenwasserstoff und anschließend mit Triphenylphosphan in Analogie zu der in EP 101597 beschriebenen Methode, liefert in guten Ausbeuten die Verbindung der Formel IV, worin X für OH steht.

In analoger Weise kann man die Verbindung der Formel I, worin R für H steht, durch Umsetzung von I mit einem Halogenierungsmittel, beispielsweise einem Halogenwasserstoff oder Phosphortrihalogenid, z.B. Phosphortribromid, und anschließend mit Triphenylphosphan in Analogie zu der in EP 101597 beschriebenen Methode in die Verbindung der Formel IV überführen, worin X für Wasserstoff steht.

Im Rahmen der Untersuchungen zur Umwandlung der Verbindung I in die Verbindung IV, worin X für OH steht, wurde überraschenderweise gefunden, dass entgegen der Lehren in der Literatur ein Entschützen der bei der Oxidation des Silylenolethers der Formel V gebildeten Verbindung der Formel VI, worin $R^s$ für eine durch Säure abspaltbare OH-Schutzgruppe, vorzugsweise für eine Acetalschutzgruppe und insbesondere für eine Gruppe $Si(R^a)_3$ steht, nicht erforderlich ist, um zur Verbindung IV mit X = Br zu gelangen. Vielmehr reicht es aus, den Silylenolether der Formel VI sukzessive mit Bromwasserstoffsäure, also einer wässrigen Lösung von Bromwasserstoff, und anschließend mit Triphenylphosphan umzusetzen, wobei man in sehr guten Ausbeuten die Verbindung der Formel IVa erhält:

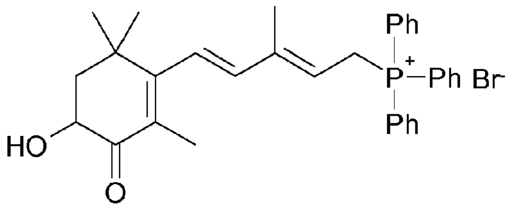
(IVa)

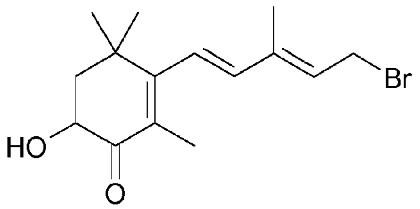
(VIa)

Eine Isolierung der bei der Umsetzung von Verbindung VI mit Bromwasserstoffsäure gebildeten Verbindung der Formel VIa ist nicht erforderlich. Vielmehr kann das bei der Umsetzung von Verbindung VI mit Bromwasserstoffsäure erhaltene Reaktionsgemisch direkt mit Triphenylphosphan umgesetzt werden. Vorzugsweise wird man vor der Umsetzung mit Triphenylphosphan überschüssigen Bromwasserstoff entfernen. Insbesondere hat es sich als vorteilhaft erwiesen, vor der Umsetzung mit Triphenylphosphan etwaige Spuren an Bromwasserstoff durch Zusatz von Alkylenoxid, wie Butylenoxid, zu entfernen (vgl. Helv. Chim. Acta 64, 2444, 1981).

Diese Vorgehensweise verkürzt die Synthese der Verbindung IVa um einen weiteren Reaktionsschritt. Dementsprechend betrifft die vorliegende Erfindung auch ein Verfahren zur Herstellung eines Phosphonium-Salzes der Formel IVa, welches die folgenden Schritte umfasst:

a") Bereitstellung eines Silylenolethers der Formel V, wie zuvor definiert, worin Rs für eine durch Säurebehandlung abspaltbare OH-Schutzgruppe steht, vorzugsweise für eine Acetalschutzgruppe oder insbesondere eine Gruppe $Si(R^a)_3$ steht, worin $R^a$ gleich oder verschieden sind und für $C_1$-$C_4$-Alkyl stehen;
b") Sukzessive Umsetzung der Verbindung der Formel V mit einer Peroxocarbonsäure und Umsetzung des Oxidationsprodukts der Formel VI mit Bromwasserstoffsäure, wobei man eine Verbindung der Formel VIa erhält:
c') Umsetzung der Verbindung der Formel VIa mit Triphenylphosphan, wobei man eine Verbindung der Formel IVa erhält.

Die Umsetzung des Oxidationsprodukts der Formel VI mit Bromwasserstoffsäure erfolgt in der Regel unter Verwendung einer 10 bis 60 gew.-%igen, insbesondere 20 bis 50 gew.-%igen wässrigen Lösung von Bromwasserstoff. Bromwasserstoff wird in der Regel im Überschuss, bezogen auf die Stöchiometrie, z.B. in einer Menge von 1,5 bis 2,5 mol pro Mol der Verbindung VI eingesetzt. Die Umsetzung von VI mit Bromwasserstoffsäure erfolgt typischerweise bei Temperaturen im Bereich von -20°C bis +25°C, insbesondere im Bereich von -10°C bis +10°C. Die bei der Umsetzung anfallende Verbindung der Formel VIa kann vor ihrer weiteren Umsetzung isoliert werden, jedoch ist dies in den meisten Fällen nicht notwendig.

Die folgenden Beispiele dienen der näheren Erläuterung der Erfindung. Hierbei werden die folgenden Abkürzungen verwendet:

HPLC: Hochleistungsflüssigkeitschromatographie
GC: Gaschromatographie
THF: Tetrahydrofuran
DMF: N,N-Dimethylformamid
DCM: Dichlormethan
NMP: N-Methylpyrrolidon
tert.-BuOOH: tert.-Butylhydroperoxid
Salen: Ligand B1
OAc: Acetat
OTf: Trifluormethansulfonat
acac: Acetylacetonat (Pentan-2,4-dionat)
OVJ: Oxovinyljonol (Verbindung der Formel I mit R = H)
tBOOVJ: tert.-Butylperoxyvinyljonol (Verbindung der Formel III mit R = H)
Eq: Äquivalent
TEA: Triethylamin
TMEDA: N,N,N',N'-Tetramethyl-1,2-ethandiamin
TBACl: Tetrabutylammoniumchlorid
TBAOH: Tetrabutylammoniumhydroxid
TBAB: Tetrabutylammoniumbromid
TBAI: Tetrabutylammoniumiodid
Triton-X: Octylphenolethoxylat
TMSCl:Trimethylsilylchlorid
TMSOMs: Trimethylsilylmethylsulfonat
DMAP: 4-(Dimethylamino)-pyridin
DBU:1,8-Diazabicyclo[5.4.0]undec-7-en

## I. Analytik

Alle Reaktionsgemische wurden mittels HPLC nach der im Folgenden beschriebenen Methode bezüglich ihrer Zusammensetzung analysiert, sofern nichts anderes angegeben ist.

| | |
|---|---|
| Gerät: | Agilent Series 1200 |
| Säule: | Ascentis Express RP-Amide 2,7µm 100*3mm von Supelco® |
| Detektor: | UV-Detektor λ=242 nm, BW=4 nm |
| Eluent: | -A: Wasser mit 0,02Vol% Ethylendiamin |
| | -B: Acetonitril |

(fortgesetzt)

| Zeit in min | %B | Flussrate |
|---|---|---|
| 0,0 | 30 | 0,6 |
| 6,0 | 70 | 0,6 |
| 7,0 | 100 | 0,6 |
| 9,0 | 100 | 0,6 |
| 9,1 | 30 | 0,6 |

Messbedingungen:

| | | | |
|---|---|---|---|
| Injektion | : 5 µL | | |
| Temperatur | : 50 °C | | |
| Laufzeit | : 12 min | | |
| Druck | : ca. 160 bar | | |

Die Kalibrierung erfolgte mit externem Standard gemäß der folgenden Vorschrift. Die Reinsubstanzen werden in folgenden Konzentrationen eingewogen:

1. 1. ca. 0,05 g / 50 ml
2. 2. ca. 0,10 g / 50 ml
3. 3. ca. 0,15 g / 50 ml
4. 4. ca. 0,20 g / 50 ml
5. 5. ca. 0,25 g / 50 ml

Die Proben wurden in Acetonitril gelöst. Die Genauigkeit der Einwaagen liegt bei 0,1 mg. Mit Hilfe eines geeigneten PC-Programms wird eine Kalibriergerade erstellt. Für die verwendeten Substanzen ist dieses eine lineare Funktion. Standardabweichung, Korrelationskoeffizient und Geradengleichung werden errechnet und sind ein Maß für die Güte der Kalibrierung. Für die Komponenten kann so ihre Konzentration, bezogen auf den jeweiligen externen Standard, ermittelt werden.

Zur Auswertung der HPLC wurde das Eluentenspektrum vom Probenspektrum abgezogen. Ausgewertet wurde der Bereich bis 11 Minuten, die restliche Laufzeit diente der Equilibrierung. Der Anteil am Edukt, in Gew.-%, berechnet sich aus der Peakfläche gemäß der folgenden Gleichung:

$$\text{Gewichtsprozent (ESTD\%)} = \frac{\text{Peakfläche x 100 x Responsefaktor}_{\text{(Substanz)}}}{\text{Einwaage(Probe)}}$$

Der Responsfaktor ist der Quotient aus Einwaage der Referenzsubstanz und Peakfläche der Referenzsubstanz.

**Beispiel 1:**

15 g Vinyljonol (96 %ig 0,068 mol) wurden in 146 g Pyridin mit 0,22 g (0,002 mol) CuCl versetzt und unter Rühren auf 35 °C erwärmt. Hierzu gab man innerhalb von 90 min. 26,3 g (0,204 mol) einer 70 gew.-%igen wässrigen Lösung von tert-Butylhydroperoxid und rührte die erhaltenen Mischung 22 h bei 35-40 °C. Quantitative HPLC des Reaktionsprodukts ergab 46,4 Gew.-% des Produkts bei vollständigem Umsatz an Edukt.

Die wässrige Phase wurde abgetrennt und noch zwei Mal mit je 60 g Toluol extrahiert. Die vereinigten organischen Phasen wurden einmal mit 50 g halbgesättigter, wässriger Natriumsulfit-Lösung und zweimal mit je 50 ml Wasser gewaschen, über $Na_2SO_4$ getrocknet und bei vermindertem Druck zur Trockne eingeengt
16.6 g Produktphase mit 38.4 % Produkt (Verbindung I, R = H).

Die so erhaltene Verbindung der Formel I (R = H) kann durch Standardreinigungsoperation wie z.B. Säulenchromatographie oder fraktionierte Destillation für die Weiterverarbeitung aufgereinigt werden.

**Beispiel 2:**

15 g Vinyljonol in 145 g Pyridin wurden mit 2,24 g Co(II)acetylacetonat (0.009 mol) versetzt. Hierzu gab man bei 40 °C innerhalb von 60 min. 26,3 g (0,204 mol) einer 70 gew.-%igen wässrigen Lösung von tert. Butylhydroperoxid. Dann erhitzte man den Reaktionsansatz 16 h auf 60 °C. Zur Vervollständigung der Reaktion erhitzte man weitere 24 h auf 70 °C. Laut HPLC enthielt das Gemisch 35,3 Gew.-% der Peroxidverbindung III (R = H, R' = tert. Butyl) und 22 Gew.-% Produkt (Verbindung I, R = H) und 8 Gew.-% Edukt (Verbindung II, R = H).

**Beispiel 3:**

In zu Beispiel 1 analoger Weise wurden 10 g Vinyljonol in 214 g Acetonitril in Gegenwart von 0,095 g CuI und 33,4 g einer 70 gew.-%igen wässrigen Lösung von tert-Butylhydroperoxid umgesetzt. Man erhielt ein Produktgemisch, dass laut HPLC 25 Gew.-% Produkt (Verbindung I, R = H) enthielt.

**Beispiel 4:**

20 g Vinyljonol und 1,56 g $CoCl_2$ wurden in 65 g Pyridin auf 60 °C erwärmt. Hierzu gab man innerhalb von 180 min. 36,6 g (3 eq.) einer 70 gew.-%igen wässrigen Lösung von tert. Butylhydroperoxid. Dann wurde der Reaktionsansatz 16 h bei 60 °C nachgerührt. Anschließend wurden weitere 36,6 g (3 eq.) einer 70 gew.-%igen wässrigen Lösung von tert. Butylhydroperoxid innerhalb von 2 h zugetropft. Laut HPLC enthielt das Gemisch 50 % des Peroxids III (R = H, R' = tert.-Butyl), sowie 3 Gew.-% Edukt (Verbindung II, R = H) und 42 Gew.-% Produkt (Verbindung I, R = H). Man rührte weitere 20 h bei 60 °C, gab weitere 12,2 g (1 eq.) einer 70 gew.-%igen wässrigen Lösung von tert. Butylhydroperoxid innerhalb von 1 h zu und rührte weitere 5 h bei 60°C nach. Laut HPLC bestand das Gemisch zu 50 Gew.-% aus dem gewünschten Produkt und zu 4,8 Gew.-% aus Peroxid.

**Beispiel 5:**

In zu Beispiel 1 analoger Weise wurden 15 g Vinyljonol in 146 g Pyridin in Gegenwart von 8,2 mmol (0,128 eq.) $CuCl_2$ und 24,7 g (3 eq.) einer 70 gew.-%igen wässrigen Lösung von tert. Butylhydroperoxid umgesetzt. Nach einer Reaktionszeit von 4 h erhielt man ein Produktgemisch, das laut HPLC 33,2 Gew.-% Produkt (Verbindung I, R = H) enthielt.

**Beispiel 6:**

In einem Reaktionsgefäß legte man 28 g Wasser und 0,114 g $RuCl_3$ vor und gab hierzu unter Rühren eine Lösung von 19,5 g Vinyljonol in 200 ml Cyclohexan. In diese Mischung gab man innerhalb von 6 h bei 15-20°C 113,9 g einer 70 gew.-%igen wässrigen Lösung von tert. Butylhydroperoxid. Der Ansatz wurde 18 h gerührt. Anschließend trennte man die organische Phase und teilte sie in zwei Portionen.

Die erste Hälfte wurde mit 100 ml gesättigter wässriger Natriumsulfit-Lösung gewaschen, über Natriumsulfat getrocknet und anschließend zur Trockne eingeengt. Laut HPLC enthielt der Rückstand 21,7 Gew.-% Produkt (Verbindung I, R = H) und 2,3 Gew.-% des Peroxids III (R = H, R' = tert.-Butyl).

Alternativ dazu wurde die zweite Hälfte mit 200 ml Wasser und 250 ml 2-Butanon versetzt. Hierzu gab man 19,5 g $Na_2SO_3$ und rührte 3 h bei 70 °C. Nach dem Abkühlen trennte man die Phasen, trocknete die organische Phase und engte sie bei 50 °C und vermindertem Druck bis zur Trockne ein. Laut HPLC enthielt der Rückstand 21,3 Gew.-% Produkt (Verbindung I, R = H) und 2,6 Gew.-% des Peroxids III (R = H, R' = tert.-Butyl).

**Beispiel 7:**

Die Isolierung des Peroxids III (R = H, R' = tert.-Butyl) erfolgte durch mehrmalige Chromatographie des Reaktionsprodukts aus Beispiel 1 an je 70 g Kieselgel mit einem Gemisch von Heptan/THF als zähes Öl.
$^1$H-NMR ($CDCl_3$, 400 MHz: 6,1-5,9 (m, 2 H), 5,6 (d, 1 H), 5,3 (d, 1 H), 5,1 (d, 1 H), 4,2 (br. S. 1 H), 2,1-2,0 m, 1 H), 1,8 (s, 3 H), 1,7-1,6 (m, 2 H), 1,6 (m, 1 H), 1,4 (s, 3 H), 1,4-1,3 (m, 1 H), 1,4 (s, 9 H), 1,0 (2 x s, 6 H).

**Beispiel 8:**

30 g Vinyljonol (0,136 mol) wurden in 97 g Pyridin mit 0,911 g $CoCl_2$ versetzt und auf 60 °C erwärmt. Innerhalb von 180 min gab man hierzu 53,8 g einer 70 gew.-%igen wässrigen Lösung von tert. Butylhydroperoxid und rührte den Ansatz 17 h bei 60 °C. Das dabei erhaltene Reaktionsgemisch enthielt 27,3 % des gewünschten Produktes (Verbindung I, R = H), 4,1 % des Eduktes und 25,7 % des Peroxids III (R = H, R' = tert.-Butyl).

Anschließend wurde das Reaktionsgemisch innerhalb von 6 h in eine auf 70 °C erwärmte Mischung von 0,397 g CuI in 74 g Pyridin getropft. Danach zeigt das HPLC 43,5 Gew.-% Produkt 1, 3,5 % des Eduktes und nur noch 1,6 % des Peroxids III. Zu der Mischung gab man 1 eq. Tert. Butylhydroperoxid und rührte den Ansatz weitere 16 h bei 60 °C. Anschließend versetzte man das Reaktionsgemisch mit 125 ml Toluol, gab hierzu 450 g Wasser und trennte die Phasen. Die organische Phase wurde einmal mit halbkonzentrierter wässriger $NaHSO_3$-Lösung und zweimal mit Wasser gewaschen und eingeengt. Auf diese Weise erhielt man 17 g Rohprodukt, das 38,7 Gew.-% des gewünschten Produkts (Verbindung I, R = H) enthielt. Dies entspricht einer Ausbeute von 43 %.

**Beispiel 10:**

0,11 mol Vinyljonol (Verbindung II, R = H) wurden mit 191 mg 1,10 Phenanthrolin und 563 mg Co(II)-(2-ethylhexanoat)$_2$ (65 gew.-%ig in Weißöl) versetzt. Der Reaktionsansatz wurde auf 60°C erwärmt. Dann gab man 45 g tert.-Butylhydroperoxid (70 % in Wasser) so zu, dass die Temperatur bei etwa 60 °C gehalten wurde. Anschließend rührte man so lange nach, bis kein weiterer Umsatz mehr zu beobachten war, setzte 19,9 g Piperdin zu und erwärmte auf 70 °C, bis sich die Konzentration des Zwischenprodukts der Formel III (R = H) nicht mehr änderte.

Zur Aufarbeitung wurde das Reaktionsgemisch mit 26 g Toluol und 50 g halbgesättigter Kochsalz-Lösung versetzt und die Phasen wurden getrennt. Die wässrige Phase wurde mit 26 g Toluol rückextrahiert und die vereinigten organischen Phasen wurden mit 50 g Wasser gewaschen. Nach Entfernung der flüchtigen Bestandteile der organischen Phase werden 30,3 g Rohprodukt mit einem Anteil von 32,7 % an Verbindung der Formel I (R = H) erhalten.

**Beispiel 11**

In Analogie zu Beispiel 11 wurden 0,10 mol Vinyljonol in 20,7 g DMF mit 556 mg Co(II)-(2-ethylhexanoat)$_2$ und 0,184 g 1,10-Phenanthrolin bei 68 bis 72 °C langsam mit 44,54 g 70 % tert-Butylhydroperoxid (in Wasser) versetzt. Hierbei wurden das Co(II)-(2-ethylhexanoat)$_2$ und das Phenanthrolin in je 2 Aliquoten vor der Zugabe des Peroxides und nach der Hälfte der Zugabe des Peroxides zugefügt. Danach wurden 16,07 g Triethylamin zugegeben und die Mischung solange bei 70 °C gerührt, bis keine Zunahme an Produkt I mehr zu beobachten war. Wässrige Aufarbeitung wie in Beispiel 10 lieferte 29,6 g Rohprodukt mit einem Gehalt an Verbindung I (R = H) von 33.1 Gew %.

**Beispiel 12:**

In Analogie zu Beispiel 10 wurden 0,1 mol Vinyljonol in 12,3 g Acetonitril und in Gegenwart von 0,8 g Co(acac)$_3$ und 0,4 g 1,10-Phenanthrolin mit 38,6 g tert.-Butylhydroperoxid umgesetzt und dann mit 20,2 g Triethylamin gerührt, bis keine weitere Produktzunahme zu beobachten war. Nach Aufarbeitung wie in Beispiel 10 wurden 23,9 g Rohprodukt erhalten, die einer auf Gew-% bezogen Ausbeute 41 % Produkt der Formel I (R = H) entsprach.

**Beispiel 13:**

0,1 mol Vinyljonol wurden in 8,4 g Pyridin gelöst. Die Lösung wurde mit 164 mg 1,3 Oxazol und 150 mg Cu(I)-Oxid versetzt und auf 40 °C erwärmt und in Analogie zu Beispiel 10 mit 45,3 g tert-Butylhydroperoxid umgesetzt. Danach wurde solange auf 70 °C erwärmt, bis keine Zunahme des Produktes der Formel I (R = H) mehr zu beobachten war. Nach Aufarbeitung wie in Beispiel 10 beschrieben, erhielt man die Verbindung der Formel I (R = H) in 32 %iger Ausbeute als Rohmischung.

**Beispiel 14**

0,3 mol Vinyljonol wurden in 70 g Pyridin mit 0,79 g Co(II)-(2-ethylhexanoat)$_2$ versetzt und bei 90 °C wurden 126 g 70-%ige, wässrige tert-Butylhydroperoxidlösung zugetropft, wobei nach Zugabe der halben Menge an tert-Butylhydroperoxidlösung weitere 0,79 g Co(II)-(2-ethylhexanoat)$_2$ zugegeben wurden. Danach wurde das Reaktionsgemisch unter Abdestillieren von Leichtsiedern auf 98 °C erhitzt, bis kein Zwischenprodukt III (R = H) mehr nachweisbar war. Nach Aufarbeitung in Analogie zu Beispiel 10 wird die Verbindung der Formel I (R = H)als Rohware in einer Ausbeute von 33 % isoliert.

**Beispiele 15a bis 15m**

1 bis 2 mol % des in der folgenden Tabelle angegebenen Metallsalzes/Komplexes wurden in 1,5 g (6,8 mmol) Vinyljonol vorgelegt und es wurden 2,92 g (22,7 mmol) 70 %ige wässrige tert.-BuOOH-Lösung in 2,7 g (34,1 mmol) Pyridin in 3 Portionen mit jeweils 1ml/min zudosiert. Die Ansätze wurden 10 h bei 40 °C nachgerührt und in der oben

beschriebenen Weise hinsichtlich der Bildung der Verbindung I (R = H) und Verbindung III (R = H) analysiert. Die Ergebnisse sind in der Folgenden Tabelle 1 zusammengefasst.

Tabelle 1:

| Beispiel | Übergangsmetallverbindung | | OVJ | tBOOVJ |
|---|---|---|---|---|
| | Metall | Verbindung | Ausbeute [%] 1) | Ausbeute [%] 1) |
| 15a | Cu(I) | $Cu_2O$ | 30,9 | 37,6 |
| 15b | Cu(I) | $[Cu(CH_3CN)_4]PF_6$ | 29,0 | 30,5 |
| 15c | Cu(II) | $CuBr_2$ | 38,8 | 14,8 |
| 15d | Cu(II) | $Cu(OAc)_2$ | 41,7 | 23,2 |
| 15e | Cu(II) | $Cu(OTf)_3$ | 34,2 | 23,7 |
| 15f | Cu(II) | $Cu(acac)_2$ x $H_2O$ | 46,6 | 5,9 |
| 15g | Co(II) | $CoBr_2$ | 16,9 | 42,8 |
| 15h | Co(II) | $CoI_2$ | 4,4 | 24,7 |
| 15i | Co(II) | $Co(BF_4)_2$ | 8,8 | 27,0 |
| 15k | Co(II) | $Co(OAc)_2$ x 4 $H_2O$ | 16,7 | 25,0 |
| 15l | Co(II) | $Co(Formiat)_2$ x 2 $H_2O$ | 7,6 | 28,8 |
| 15m | Co(II) | Co-Phtalocyanin | 21,4 | 14,1 |
| 1) Ausbeute bezogen auf eingesetztes Vinyljonol | | | | |

**Beispiele 16a bis 16v**

In Analogie zu Beispiel 11 wurde Vinyljonol in DMF oder NMP zuerst mit tBuOOH umgesetzt. Das dabei erhaltene Reaktions wurde dann entsprechend den Angaben in Tabelle 2 mit einer Base zum Endprodukt der Formel I (R = H) weiter umgesetzt. Die Ausbeuten sind in Tabelle 2 angegeben:

Tabelle 2:

| Bsp. | Temp. | Base / Menge 1) | Zeit [h] | Ausb. OVJ 2) |
|---|---|---|---|---|
| 16a | 70 °C | Piperidin / 1,0 Eq | 19 | 41,5% |
| 16b | 70 °C | TEA /1,0 Eq | 19 | 43,3% |
| 16c | 70 °C | Morpholin / 1,0 Eq | 19 | 34,1% |
| 16d | 70 °C | Na-Methylat /1,0 Eq | 19 | 41,2% |
| 16e | 70 °C | wässrige NaOH 50 % / 1,0 Eq | 19 | 40,8% |
| 16f | 70 °C | wässrige KOH 50 % / 1,0 Eq | 19 | 40,3% |
| 16g | 70 °C | Kalium-tert-butylat / 1,0 Eq | 19 | 40,1% |
| 16h | 70 °C | $NaHCO_3$ / 1,0 Eq | 19 | 28,8% |
| 16i | 70 °C | $Na_2CO_3$ / 1,0 Eq | 19 | 26,7% |
| 16k | 70 °C | TEA / 1,1 Eq | 16 | 43,0% |
| 16l | 70 °C | TEA / 2 Eq | 23 | 43,6% |
| 16m | 70 °C | Piperidin / 1,0 Eq | 12 | 43,2% |
| 16n | 70 °C | Tripropylamin / 2 eq | 12 | 40.3% |
| 16o | 70 °C | TMEDA / 1 Eq | 12 | 34.4% |
| 16p | 70°C | N,N-Dimethylethanolamin / 2 Eq | 12 | 35.6% |

(fortgesetzt)

| Bsp. | Temp. | Base / Menge 1) | Zeit [h] | Ausb. OVJ 2) |
|---|---|---|---|---|
| 16q | 70°C | 2-Methylaminoethanol / 2eq | 12 | 35.6% |
| 16r | 70°C | NaOH 25%/ 1,0 Eq | 5 | 22.7% |
| 16s | 70°C | NaOH 50 % / 1,0 Eq 20 mol% Triton-X | 12 | 35.3% |
| 16t | 70°C | NaOH 50%ig 1eq 20 mol% TBACI | 12 | 32.5% |
| 16u | 70°C | NaOH 50%ig / 1eq 20 mol% TBAOH | 12 | 32.2% |
| 16v | 70°C | NaOH 50%ig / 1eq 10 mol% TBACI | 12 | 33.9% |
| 1) Mengenangaben auf Vinyljonol bezogen<br>2) Ausbeute an Oxovinyljonol, bezogen auf Vinyljonol | | | | |

**Analytik für Verbindung V**

Alle Rohausträge zur Herstellung von V wurden mittels Gaschromatographie (GC) nach der im Folgenden beschriebenen Methode bezüglich ihrer Zusammensetzung analysiert, sofern keine weiteren Angaben gemacht wurden.

Geräteeinstellungen und chromatographische Bedingungen:

| | |
|---|---|
| Gerät: | Agilent 6890 N |
| Trägergas: | Stickstoff |
| Trennsäule: | Chrompack / 50 m CP- Sil 5 / ID = 0,25 mm, FD = 0,12 μm |
| Injektionssystem: | HP-Split / Splitless Injektor / Modus Split 1 : 109 |
| Injektion: | HP-GC Injector 7683 Serie / Menge = 1 μl |
| Detektion: HP- FID | |

Temperaturen + Drücke:

| | |
|---|---|
| Detektor: | 300°C |
| Injektor: | 250°C |
| Starttemp.: | 50°C |
| Verweilzeit 1: | 0 min |
| Rate 1: | 10°C / min |
| Endtemp. 1: | 300°C |
| Verweilzeit 2: | 15 min |
| Laufzeit total: | 40 min |
| Druck (prgm): | 10,77 PSI konstant |
| Septumpurge: | 0,6 ml / min |

**Probenvorbereitung:**

Die Proben sind ca. 20 % ig gelöst. Lösungsmittelunterdrückung von 0 - 8 min.

**Auswertung / Software:**

$$\text{Flächenprozent (N \%)} = \frac{\text{Peakfläche(iA)} \times 100}{\text{Gesamtfläche (A)}}$$

| Retentionszeit: | Oxovinyljonol (Formel I; R = Wasserstoff) = 22.461 min |
|---|---|
| | Silylenolether (Formel V; $R^a = CH_3$, $R^s = Si(CH_3)_3$) = 24.030 min |

**Beispiel 17 (Herstellungsbeispiel für Verbindung VI, $R^a = CH_3$, $R^s = Si(CH_3)_3$):**

Nach Aufreinigung durch Säulenchromatographie an Kieselgel (Essigester/Heptan) wurde die Verbindung aus Beispiel 1 in Analogie zu Beispiel 6 der EP 101597 in den Silylenolether der Formel V ($R^a = CH_3$, $R^s = Si(CH_3)_3$) überführt. Dieser wurde in Analogie zu Beispiel 8, Schritt a) der EP 101597 in die Verbindung der Formel VI ($R^a = CH_3$, $R^s = Si(CH_3)_3$) überführt

**Beispiele 18a bis 18l**

**Beispiele zur Herstellung des Silylenolether der Formel V (1 - 5 g der Verbindung I)**

Die Verbindung der Formel I (R = H) wurde im angegebenen Lösungsmittel mit der Aminbase (und eventuell einem weiteren Zusatz z.B. einem Phasentransferkatalysator) in den in der Tabelle 3 aufgeführten Verhältnissen (bezogen auf den Reinstoff der Verbindung I) bei Raumtemperatur gelöst. Im Anschluss wurde das entsprechende Silylierungsreagenz, mit den in der Tabelle 3 angegebenen Äquivalenten (bezogen auf den Reinstoff der Verbindung I) innerhalb von 1-2 Minuten zugetropft und das Gemisch wurde für weitere 10 Minuten bei Raumtemperatur gerührt. Danach wurde das Reaktionsgemisch über 16 h bei Rückfluss gerührt, bis kein weiterer Umsatz mehr detektiert werden konnte. Für die analytische Untersuchung (GC FI.-%) wurde dem Gemisch eine Probe (2,5 ml) entnommen, mit Toluol (2,5 ml) und Eiswasser (2,5 ml) versetzt und in der Kälte gewaschen. Nach der Phasentrennung wurde die organische Phase über $Na_2SO_4$ getrocknet und das Filtrat gaschromatographisch untersucht.

Tabelle 3:

| Bsp. | Reagenz | Eq 1) | Base | Eq 1) | Lösungmittel [g/g]2 | V 3) | Bemerkung 4) |
|---|---|---|---|---|---|---|---|
| 18a | TMSCI | 6,8 | TEA | 8,7 | DMF 1,4 | 90,2 | - |
| 18b | TMSCI | 6,2 | TEA | 7,9 | DMF 1,3 | 92,0 | Zusatz von je 0,05 Eq. TBAB und DMAP |
| 18c | TMSCI | 6,6 | TEA | 8,4 | Acetonitril 1,4 | 92,2 | - |
| 18d | TMSCI | 5,0 | TEA | 6,7 | Acetonitril 1,3 | 91,7 | Zusatz von 0,05 Äq. TBAB |
| 18e | TMSCI | 5,1 | TEA | 6,3 | Acetonitril 1,3 | 92,6 | Zusatz von 0,05 Äq. TBAB |
| 18f | TMSCI | 5,1 | TEA | 6,3 | Acetonitril 1,3 | 92,9 | Zusatz von 0,05 Äq. TBAI |
| 18g | TMSOMs | 8,2 | TEA | 10,5 | Acetonitril 1,7 | 78,0 | - |
| 18h | TMSOMs | 6,7 | TEA | 9,8 | Acetonitril 1,7 | 79,2 | - |
| 18i | TMSOMs | 6,4 | TEA | 8,1 | DCM 1,3 | 87,2 | - |
| 18k | TMSOMs | 6,5 | TEA | 8,2 | DCM 1,3 | 87,8 | Zusatz von 5 mol% DMAP |
| 18l | TMSOMs | 6,5 | TEA | 8,2 | DCM 1,3 | 87,3 | Zusatz von 5 mol% DBU |
| 1) | Äquivalente bezogen auf die Stoffmenge der Verbindung der Formel I (R = H) | | | | | | |
| 2) | Lösungsmittelmenge in g pro g Oxovinyljonol | | | | | | |

(fortgesetzt)

| Bsp. | Reagenz | Eq 1) | Base | Eq 1) | Lösungmittel [g/g]2 | V 3) | Bemerkung 4) |
|---|---|---|---|---|---|---|---|
| 3) | in GC-FI.-% | | | | | | |
| 4) | Mengenangaben, bezogen auf Oxovinyljonol | | | | | | |
| Bei der Verwendung von TMSOMs in Gegenwart entsprechender Aminbasen und in Abhängigkeit des verwendeten Lösungsmittels wurde nach Reaktionsende und Abkühlphase vor der wässrigen Aufarbeitung, eine flüssige Unterphase (Ammonium-Mesilat), abgetrennt. | | | | | | | |

**Herstellung des Silylenolethers (V) und dessen Oxidation:**

**HPLC-Analytik für die Oxidationsprodukte VI und VIIa**

| | | |
|---|---|---|
| Gerät: | Agilent Series 1100 | |
| Säule: | Zorbax Eclipse XDB C18 1,8μm 50*4,6mm von Agilent® | |
| Eluent: | -A: Wasser mit 0,05Vol% Triethylamin | |
| | -B: Tetrahydrofuran | |
| Zeit in min | %B | Fluss |
| 0,0 | 15 | 1,2 |
| 6,0 | 60 | 1,2 |
| 8,0 | 100 | 1,2 |
| 10,0 | 100 | 1,2 |
| 10,1 | 15 | 1,2 |
| Detektor: | UV-Detektor λ=250 nm, BW=5 nm | |
| Flussrate: | 1,2 ml/min | |
| Injektion: | 5 μL | |
| Temperatur: | 50°C | |
| Laufzeit: | 12 min | |
| Druck: | ca. 200 bar | |

**Probenvorbereitung:**

Eine entsprechende Menge Probe wurde in Tetrahydrofuran gelöst.
Bei Reinsubstanzen wurde eine Einwaage von 15 mg auf 10 ml Lösemittel gewählt, um eine gute Auflösung der Verunreinigungen zu erhalten.
Proben die sich in Tetrahydrofuran und/oder Wasser nicht vollständig lösten, wurden filtriert.

**Auswertung / Software :**

Das Eluentenspektrum wurde vom Probenspektrum abgezogen.
Ausgewertet wurde der Bereich bis 10 Minuten, die restliche Laufzeit diente nur dazu das System, wieder zu equilibrieren.

$$\text{Flächenprozent (A\%)} = \frac{\text{Peakfläche}}{\text{Gesamtfläche}} \times 100\%$$

| Retentionszeit: | Verbindung der Formel VI ($R^a = CH_3$, $R^s = Si(CH_3)_3$) = 7.474 min |
|---|---|
| | Verbindung der Formel VIIa ($R^s = Si(CH_3)_3$) = 5.898 min |

**Beispiel 19:**

23,0 g (136,6 mmol) Bromwasserstoffsäure (48 Gew.-%) wurden bei -5 °C vorgelegt. Hierzu gab man eine Lösung von 27,3 g der Verbindung der Formel VI aus Beispiel 17 in 240 ml Dichlormethan bei -5 bis 0 °C und rührte anschließend 40 min bei 0 °C. Anschließend wurde das Reaktionsgemisch mit Eiswasser (36 ml) versetzt. Nach Abtrennung der wässrigen Phase wurde die organische Phase mit 38 g wässriger $NaHCO_3$-Lösung (1,25 %ig) gewaschen. Die organische Phase wurde nochmals mit Wasser (43 ml) gewaschen. Im Anschluss wurde die erhaltene Lösung bei Raumtemperatur mit 0,33 ml Butylenoxid (= 1,2-Epoxybutan) versetzt. Anschließend tropfte man eine Lösung von Triphenylphosphan (17,9 g, 68,3 mmol) in Toluol (26 ml) zu und versetzte den Ansatz erneut mit Butylenoxid (0,33 ml). Nach einer Reaktionszeit von 1 h bei Raumtemperatur wurde die Reaktionslösung auf Rückfluss erhitzt und ein Lösungsmitteltausch von Dichlormethan gegen Toluol wurde bei Normaldruck durchgeführt. Die Lösung wurde gekühlt (0 - 5 °C), das dabei anfallende Kristallisat wurde abgesaugt, mit Toluol gewaschen und über Nacht im $N_2$-Strom getrocknet. Man erhielt 27,4 g des Phosphoniumbromids der Formel IVa als hell-ockerfarbenen Feststoff (Schmelzpunkt 167-169°C).

**Beispiel 20**

Unter Rühren wurde Verbindung der Formel I (167,5 mmol) in Acetonitril (80.0 ml), Triethylamin (177,08 g) und Tetrabutylammoniumbromid (4,03 g) bei Raumtemperatur vorgelegt. Im Anschluss wurde zum Gemisch 149,38 g TMSCI innerhalb von 45 min bei 20 - 25 °C zugetropft. Nach Beendigung der Zugabe rührte man den Ansatz noch weitere 5 min bei 20 - 25 °C und erhitzte dann die erhaltene Suspension 24 h auf Rückfluss. Man kühlte auf 0 °C ab und versetzte das Gemisch mit 750 ml Toluol. Im Anschluss wurde innerhalb von 1 h bei 0 - 5 °C Wasser (250 ml) zugetropft. Nach Beendigung der Wasserzugabe wurden weitere 15 min gerührt, bevor die Phasen getrennt wurden. Anschließend wurde die Oberphase 3x mit je 250 ml Wasser bei 0 - 5 °C gewaschen. Die toluolische Phase wurde unter Rühren bei 0 °C nacheinander mit 27.08 g wasserfreiem Magnesiumsulfat und 26.25 g Natriumhydrogencarbonat versetzt. Im Anschluss wurden bei 0 - 5 °C innerhalb von 2 h insgesamt 97,5 g Peressigsäure (wässrig, 39 gew.-%ig) zugetropft. Nach vollständigem Umsatz (lt. Dünnschichtchromatographie; Cyclohexan: Essigsäureethylester 20:1) wurde das Reaktionsgemisch unter kräftigem Rühren bei 0 °C mit insgesamt 500 ml Wasser versetzt und 10 min gerührt. Nach der Phasentrennung wurde die Oberphase nacheinander zweimal mit 20 gew.-%iger, wässriger Natriumdisulfit-Lösung (je 250 ml), einmal mit 250 ml Wasser und einmal mit 2,5 gew.-%iger, wässriger Natriumhydrogencarbonat-Lösung gewaschen. Die organische Phase wurde über Natriumsulfat getrocknet, filtriert und im Vakuum (50 °C, bis 10 mbar) eingeengt. Das Rohprodukt (87,94 g) lag als ein Gemisch der Verbindungen der Formeln VI (68,0 Fl.-%) und VIIa (11,6 Fl.-%) als ein hellbraunes niederviskoses Öl vor. Das Verhältnis der Fl.-% von VI und VIIa ist aufgrund des unterschiedlichen Fortschritts der Hydrolyse der Silylschutzgruppe im Allgemeinen variabel, und kann bspw. nach längerer Standzeit im Verhältnis 1 (Verbindung der Formel VI) : 4 (Verbindung der Formel VIIa) vorliegen. Das erhaltene Rohprodukt wurde direkt in folgende Synthese eingesetzt.

Unter kräftigem Rühren legte man 63,22 g wässrige HBr (48 %ig) bei 0 °C vor und tropfte hierzu innerhalb von 30 min das Rohprodukt der vorangegangenen Stufe als Gemisch der Verbindungen der Formeln VI und VIIa (87,94 g) in 700 ml Methylenchlorid. Nach vollständigem Umsatz (lt. Dünnschichtchromatographie; Cyclohexan: Essigsäureethylester 20:1) wurde das Reaktionsgemisch bei 0 °C mit 120 ml Wasser versetzt. Nach der Phasentrennung wurde die organische Phase nacheinander mit 2,5 %iger Natriumhydrogencarbonat-Lösung (120 ml) und Wasser (120 ml) bei 0 - 5 °C gewaschen.

Die organische Phase wurde bei 20 - 25 °C vorgelegt und unter Rühren mit 1,2-Epoxybutan (901 mg) versetzt. Anschließend wurde eine Lösung von Triphenylphosphin (53,77 g) in 100 ml Toluol tropfenweise zugegeben. Nach vollständiger Zugabe wurde das Reaktionsgemisch insgesamt 1 h gerührt. Das Gemisch wurde erneut mit 1,2-Epoxybutan (901 mg) versetzt. Das Reaktionsgemisch wurde auf Rückfluss erhitzt und ein Lösungsmitteltausch von Dichlormethan gegen Toluol (1500 ml) bei Normaldruck durchgeführt. Die Lösung wurde auf 0 - 5 °C abgekühlt und für 1 h bei dieser Temperatur gerührt. Das Kristallisat wurde abgesaugt und 3x mit je 150 ml Toluol gewaschen. Der Rückstand wurde über Nacht im $N_2$-Strom getrocknet und lieferte 77 g des Phosphoniumbromids IVa (84,29 HPLC-Gew.%) als hell-ockerfarbenen Feststoff.

**HPLC-Analytik für Phosphoniumbromid IVa**

Die Phosphonium-Salze IVa wurden mittels HPLC nach der im Folgenden beschriebenen Methode bezüglich ihrer Zusammensetzung analysiert, sofern nichts anderes angegeben ist.

| | | |
|---|---|---|
| Gerät: | Agilent Series 1100 | |
| Säule: | LiChrospher® 60 RP-select B 5μm 250 x 4mm | |
| Eluent: | Gradientenfahrweise $KH_2PO_4$-Lösung und Acetonitril | |
| | A = Acetonitril | |
| | B = 10 mMol $KH_2PO_4$-Lösung pH=2,5 (eingestellt mit 85%iger $H_3PO_4$) | |
| Zeit | % B | Flow |
| 0.00 | 60 | 0.8 |
| 20.00 | 30 | 0.8 |
| 40.00 | 30 | 0.8 |
| Detektor: | | DAD: λ=220 nm, Bandweite 4 |
| Flussrate: | | 0,8 ml/min |
| Injektion: | | 20 μL |
| Temperatur: | | 30°C ± 0.5°C |
| Laufzeit: | | 45 min |
| Druck: | | ca. 90 bar |

**Kalibrierung:**

Die Kalibrierung erfolgte mit Hilfe von externen Standards.
Die Reinsubstanz Phosphoniumbromid IVa wurde in folgenden Mengen in einem 50 ml Kolben eingewogen:

1. 1. 5 mg
2. 2. 10 mg
3. 3. 20 mg
4. 4. 30 mg
5. 5. 40 mg

Die Proben wurden gelöst in 50Vol% Eluent B und 50Vol% Wasser (2 Minuten Ultraschall) und dann aufgefüllt. Die Genauigkeit der Einwaagen lag bei 0,1mg. Mit Hilfe eines geeigneten PC-Programms wurde eine Kalibriergerade erstellt. Für die oben aufgeführte Substanz ist dieses eine lineare Funktion. Standardabweichung, Korrelationskoeffizient und Geradengleichung wurden errechnet und sind ein Maß für die Güte der Kalibrierung. Für die Komponente konnte so ihre Konzentration, bezogen auf den jeweiligen externen Standard, ermittelt werden.

**Probenvorbereitung:**

Die Substanz wurde in entsprechender Konzentration in 50Vol% Eluent B und 50 Vol% Wasser in einem 50 ml Kolben gelöst. Die so vorbereitete Probe konnte direkt vermessen werden.

**Auswertung / Software** :

$$\text{Gewichtsprozent (ESTD\%)} = \frac{\text{Peakfläche} \times 100 \times \text{Responsefaktor}_{\text{(Phosphoniumsalz)}}}{\text{Einwaage(Probe)}}$$

$$\text{Responsefaktor}_{(\text{Phosphoniumsalz})} = \frac{\text{Einwaage (Phosphoniumsalz)}}{\text{Fläche (Phosphoniumsalz)}}$$

| Retentionszeit: | Verbindung der Formel IVa : |
|---|---|
| | Isomer 1: 13.960 min; Isomer 2: 14.807 min |

**Patentansprüche**

**1.** Verfahren zur Herstellung von Oxovinyljonol und dessen O-geschützten Derivaten der Formel I

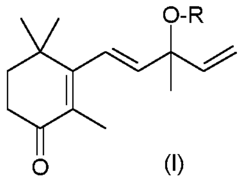

(I)

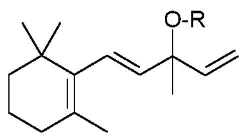

(II)

worin R für Wasserstoff oder eine OH-Schutzgruppe steht, **dadurch gekennzeichnet, dass** man β-Vinyljonol oder ein O-geschütztes Derivat davon der Formel II, worin R die zuvor genannten Bedeutungen aufweist, mit einem Oxidationsmittel in Gegenwart wenigstens eines Übergangsmetalls umsetzt, welches ausgewählt ist unter Cu, Co, Fe und Mn und deren Mischungen und wobei das Oxidationsmittels wenigstens eine sauerstoffhaltige Verbindung umfasst, die unter organischen Hydroperoxiden ausgewählt ist.

**2.** Verfahren nach Anspruch 1, **dadurch gekennzeichnet, dass** das Oxidationsmittel ein organisches Hydroperoxid umfasst, das ausgewählt ist unter Alkylhydroperoxiden und Arylalkylhydroperxoiden.

**3.** Verfahren nach einem der vorhergehenden Ansprüche, **dadurch gekennzeichnet, dass** man das Oxidationsmittel in einer Menge von 1 bis 10 mol pro mol der Verbindung der Formel II einsetzt.

**4.** Verfahren nach einem der vorhergehenden Ansprüche, **dadurch gekennzeichnet, dass** man das Übergangsmetall in Form einer Übergangsmetallverbindung einsetzt.

**5.** Verfahren nach einem der vorhergehenden Ansprüche, **dadurch gekennzeichnet, dass** die Umsetzung mit dem Oxidationsmittel in Gegenwart eines Komplexliganden, der wenigstens ein zur Koordination mit dem Übergangsmetall geeignetes Stickstoffatom aufweist, durchgeführt wird.

**6.** Verfahren nach einem der vorhergehenden Ansprüche, **dadurch gekennzeichnet, dass** man die Umsetzung in einem organischen Lösungsmittel oder in einer Mischung davon mit Wasser durchführt.

**7.** Verfahren nach einem der vorhergehenden Ansprüche, **dadurch gekennzeichnet, dass** die Variable R in den Formeln I und II Wasserstoff bedeutet.

**8.** Verfahren nach einem der vorhergehenden Ansprüche, wobei man bei der Umsetzung mit dem Oxidationsmittel zunächst eine Verbindung der allgemeinen Formel III herstellt:

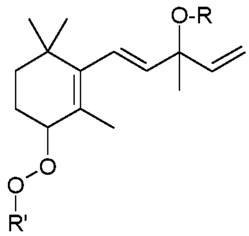

(III)

worin R für Wasserstoff oder eine OH-Schutzgruppe steht, und R' für Wasserstoff oder den von dem organischen Hydroperoxid abgeleiteten Rest steht und insbesondere Wasserstoff, Alkyl oder Arylalkyl bedeutet; und anschließend die Verbindung der Formel (III) zur Verbindung der Formel I zersetzt.

**9.** Verfahren nach Anspruch 8, wobei man die Verbindung der Formel III thermisch oder durch Behandlung mit einer Base zersetzt.

**10.** Verbindung der allgemeinen Formel III

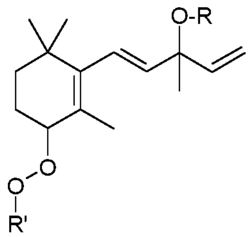

(III)

worin R für Wasserstoff oder eine OH-Schutzgruppe steht, und R' für tertiäres $C_4$-$C_8$-Alkyl oder für Phenyl-$C_1$-$C_8$-alkyl steht, wobei Phenyl unsubstituiert ist oder 1, 2 oder 3 $C_1$-$C_4$-Alkylgruppen trägt steht.

**11.** Verfahren zur Herstellung von Phosphonium-Salzen der Formel IV

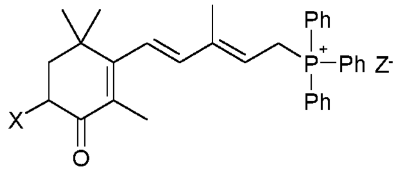

(IV)

worin X für Wasserstoff, OH oder OR" steht, Ph für Phenyl steht, $Z^-$ ein Halogenid-Anion bedeutet und R" für Wasserstoff oder eine OH-Schutzgruppe steht, umfassend:

i) Bereitstellung von Oxovinyljonol oder eines O-geschützten Derivats davon der Formel I durch ein Verfahren gemäß einem der Ansprüche 1 bis 5 oder 7;
ii) Umwandlung von Oxovinyljonols oder seines O-geschützten Derivats der Formel I in an sich bekannter Weise in eine Verbindung der Formel IV.

**12.** Verfahren nach Anspruch 11 zur Herstellung einer Phosphonium-Verbindung der Formel IV, worin X für OH steht, wobei Schritt ii) die folgenden Stufen umfasst:

a) Überführung einer Verbindung der Formel I in einen Silylenolether der Formel V

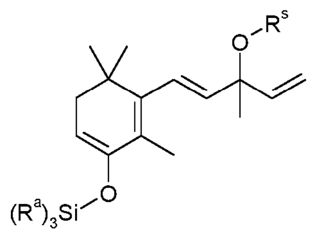

(V)

worin $R^a$ in der Gruppe $Si(R^a)_3$ gleich oder verschieden sein können und unabhängig voneinander für $C_1$-$C_4$-Alkyl stehen und $R^s$ für eine durch Säure abspaltbare OH-Schutzgruppe steht;

b) Sukzessive Umsetzung der Verbindung der Formel V mit einer Peroxocarbonsäure und gegebenenfalls anschließende Hydrolyse der dabei erhaltenen Verbindung der Formel VI, worin $R^a$ und $R^s$ die zuvor genannten Bedeutungen aufweisen, wobei man eine Verbindung der Formel VII erhält:

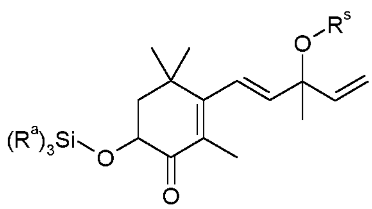

(VI)

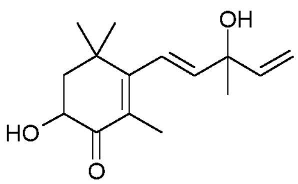

(VII)

c) Sukzessive Umsetzung der Verbindung der Formel VII mit einem Halogenierungsmittel und anschließend mit Triphenylphosphan, wobei man eine Verbindung der Formel IV erhält, worin X für OH steht,

oder

a') Umwandlung einer Verbindung der Formel I, worin R für eine durch Säure abspaltbare OH-Schutzgruppe $R^s$ steht, in ihr Enolat,

b') Umsetzung des Enolats mit einem N-Arylsulfonyloxaziridin, wobei man eine Verbindung der Formel VIa erhält

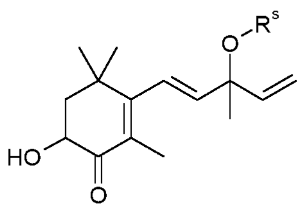

(VIa)

c) Hydrolyse der Verbindung der Formel VIa, und sukzessive Umsetzung des Hydrolyseprodukts mit einem Halogenierungsmittel und anschließend mit Triphenylphosphan, wobei man eine Verbindung der Formel IV erhält, worin X für OH steht.

**13.** Verfahren nach einem der Anspruche 11 bis 12 zur Herstellung einer Phosphonium-Verbindung der Formel III, worin X für H steht, wobei Schritt ii) die folgende Stufe umfasst:
c') Sukzessive Umsetzung der Verbindung der Formel 1, worin R für Wasserstoff steht, mit Halogenwasserstoff HZ und anschließend mit Triphenylphosphan, wobei man eine Verbindung der Formel IV erhält.

**14.** Verfahren nach Anspruch 11 zur Herstellung eines Phosphonium-Salzes der-Formel IVa

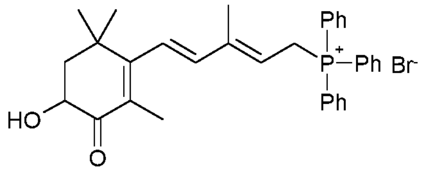

(IVa)

worin Ph für Phenyl steht, umfassend:

a") Bereitstellung eines Silylenolethers der Formel V

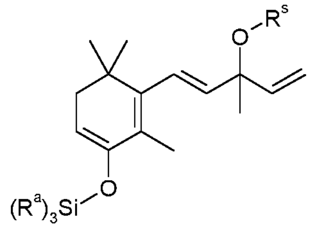

(V)

worin $R^a$ in der Gruppe $Si(R^a)_3$ gleich oder verschieden sein können und unabhängig voneinander für $C_1$-$C_4$-Alkyl stehen und $R^s$ für eine durch Säure abspaltbare OH-Schutzgruppe steht;
b") Sukzessive Umsetzung der Verbindung der Formel V mit einer Peroxocarbonsäure und Umsetzung des Oxidationsprodukts mit Bromwasserstoffsäure, wobei man eine Verbindung der Formel VIa erhält:

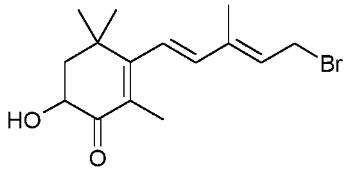

(VIa)

c') Umsetzung der Verbindung der Formel VIa mit Triphenylphosphan, wobei man eine Verbindung der Formel IVa erhält.

**15.** Verfahren nach Anspruch 14, wobei die Bereitstellung der Verbindung der Formel V die folgenden Schritte umfasst:

i) Bereitstellung von Oxovinyljonol oder eines O-geschützten Derivats davon der Formel I durch ein Verfahren gemäß einem der Ansprüche 1 bis 5 oder 7;
ii') Überführung einer Verbindung der Formel I in einen Silylenolether der Formel V.

**Claims**

**1.** A process for the preparation of oxovinylionol and its O-protected derivatives of the formula I

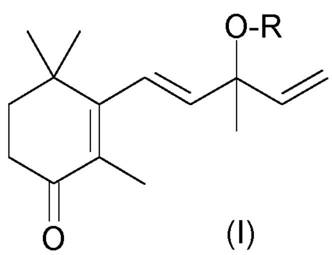

(I)

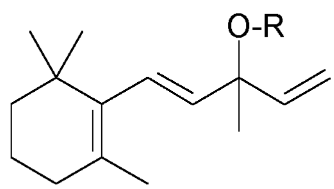

(II)

in which R is hydrogen or an OH protecting group, wherein β-vinyl ionol or an O-protected derivative thereof of the formula II in which R has the abovementioned meanings is reacted with an oxidant in the presence of at least one

transition metal which is selected from among Cu, Co, Fe and Mn and their mixtures and where the oxidant comprises at least one oxygen-containing compound selected from among organic hydroperoxides.

**2.** The process according to claim 1, wherein the oxidant comprises an organic hydroperoxide which is selected from among alkyl hydroperoxides and arylalkyl hydroperoxides.

**3.** The process according to either of the preceding claims, wherein the oxidant is employed in an amount of from 1 to 10 mol per mole of the compound of the formula II.

**4.** The process according to any of the preceding claims, wherein the transition metal is employed in the form of a transition metal compound.

**5.** The process according to any of the preceding claims, wherein the reaction with the oxidant is carried out in the presence of a complex ligand which has at least one nitrogen atom suitable for coordination with the transition metal.

**6.** The process according to any of the preceding claims, wherein the reaction is carried out in an organic solvent or in a mixture thereof with water.

**7.** The process according to any of the preceding claims, wherein the variable R in formulae I and II is hydrogen.

**8.** The process according to any of the preceding claims, wherein, during the reaction with the oxidant, a compound of the general formula III is firstly prepared:

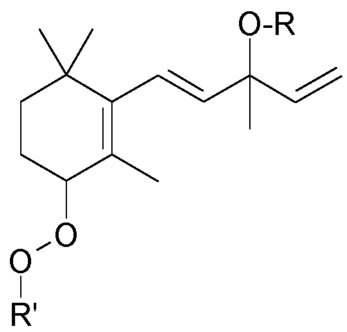

(III)

in which R is hydrogen or an OH protecting group, and R’ is hydrogen or the radical derived from the organic hydroperoxide and is in particular hydrogen, alkyl or arylalkyl;
and then the compound of the formula (III) is decomposed to give the compound of the formula I.

**9.** The process according to claim 8, wherein the compound of the formula III is decomposed thermally or by treatment with a base.

**10.** A compound of the general formula III

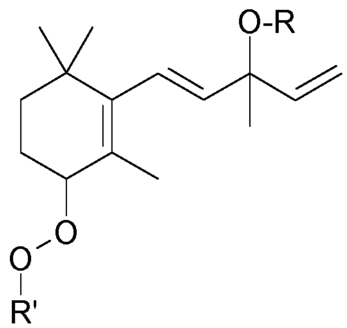

(III)

in which R is hydrogen or an OH protecting group, and R’ is tertiary $C_4$-$C_8$-alkyl or is phenyl-$C_1$-$C_8$-alkyl, where phenyl is unsubstituted or carries 1, 2 or 3 $C_1$-$C_4$-alkyl groups.

**11.** A process for the preparation of the phosphonium salts of the formula IV

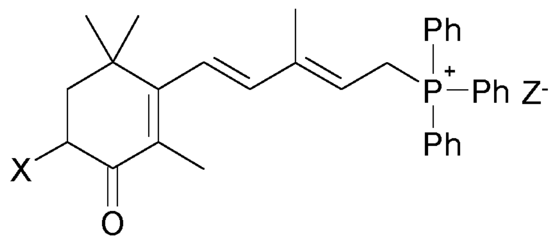

(IV)

in which X is hydrogen, OH or OR'', Ph is phenyl, $Z^-$ is a halide anion and R' ' is hydrogen or an OH protecting group, which comprises the following:

i) providing oxovinylionol or an O-protected derivative thereof of the formula I by a process according to any of claims 1 to 5 or 7;
ii) converting oxovinylionol or its O-protected derivative of the formula I in a manner known per se to give a compound of the formula IV.

**12.** The process according to claim 11 for the preparation of a phosphonium compound of the formula IV in which X is OH, wherein step ii) comprises the following stages:

a) converting a compound of the formula I into a silyl enol ether of the formula V

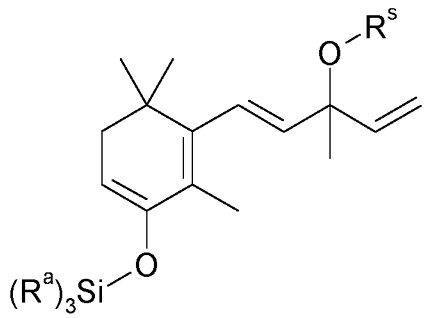

(V)

in which $R^a$ in the group $Si(R^a)_3$ can be identical or different and independently of one another are $C_1$-$C_4$-alkyl and $R^s$ is an acid-cleavable OH protecting group;
b) successively converting the compound of the formula V with a peroxocarboxylic acid and optionally subsequently hydrolyzing the resulting compound of the formula VI in which $R^a$ and $R^s$ have the abovementioned meanings, which gives a compound of the formula VII:

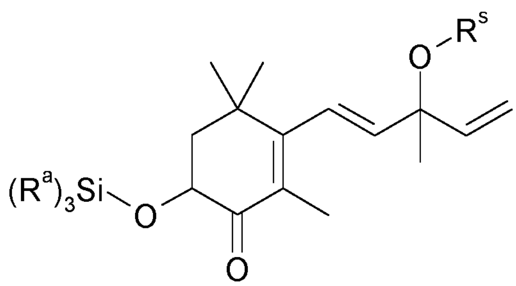

(VI)

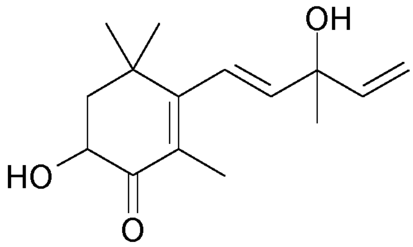

(VII)

c) successively reacting the compound of the formula VII with a halogenating agent and subsequently with triphenylphosphane, which gives a compound of the formula IV in which X is OH,

or

a') converting a compound of the formula I, in which R is an acid-cleavable OH protecting group $R^s$, into its enolate,
b') reacting the enolate with an N-arylsulfonyloxaziridine, which gives a compound of the formula VIa

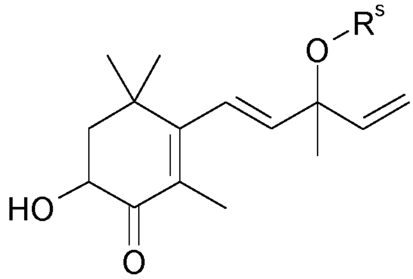

(VIa)

,

c) hydrolyzing the compound of the formula VIa and successively reacting the hydrolyzate with a halogenating agent and subsequently with triphenylphosphane, which gives a compound of the formula IV in which X is OH.

**13.** The process according to any of claims 11 to 12 for the preparation of a phosphonium compound of the formula IV in which X is H, wherein step ii) comprises the following stage:
c') successively reacting the compound of the formula I in which R is hydrogen with hydrogen halide HZ and subsequently with triphenylphosphane, which gives a compound of the formula IV.

**14.** The process according to claim 11 for the preparation of a phosphonium salt of the formula IVa

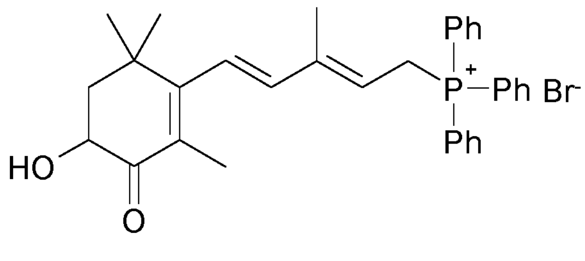

(IVa)

in which Ph is phenyl, which comprises:

a'') providing a silyl enol ether of the formula V

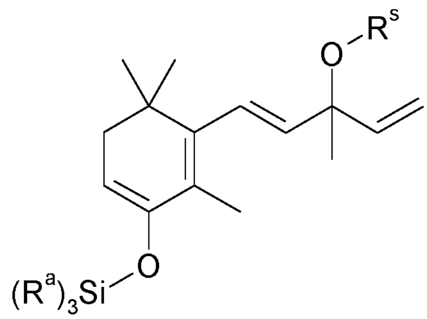

(V)

in which $R^a$ in group $Si(R^a)_3$ can be identical or different and independently of one another are $C_1$-$C_4$-alkyl and $R^s$ is an acid-cleavable OH protecting group;
b'') successively reacting the compound of the formula V with a peroxo carboxylic acid and reacting the oxidation product with hydrobromic acid, which gives a compound of the formula VIa:

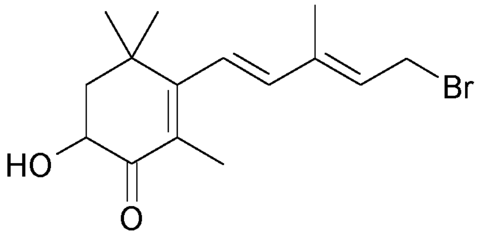

(VIa)

c') reacting the compound of the formula VIa with triphenylphosphane, which gives a compound of the formula IVa.

**15.** The process according to claim 14, wherein providing the compound of the formula V comprises the following steps:

i) providing oxovinylionol or an O-protected derivative thereof of the formula I by a process according to any of claims 1 to 5 or 7;
ii') converting a compound of the formula I into a 5 silyl enol ether of the formula V.

**Revendications**

**1.** Procédé de fabrication d'oxovinylionol et de ses dérivés O-protégés de formule I

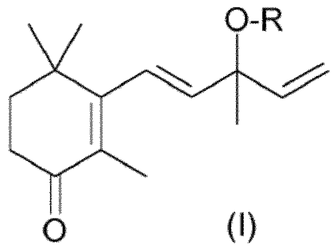

(I)

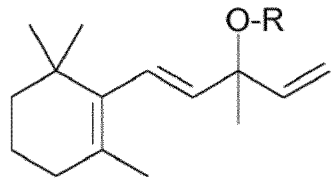

(II)

dans laquelle R représente l'hydrogène ou un groupe protecteur OH, **caractérisé en ce que** du β-vinylionol ou un dérivé O-protégé de celui-ci de formule II, dans laquelle R a les significations indiquées précédemment, est mis en réaction avec un oxydant en présence d'au moins un métal de transition, qui est choisi parmi Cu, Co, Fe et Mn et leurs mélanges, l'oxydant comprenant au moins un composé contenant de l'oxygène, qui est choisi parmi les hydroperoxydes organiques.

**2.** Procédé selon la revendication 1, **caractérisé en ce que** l'oxydant comprend un hydroperoxyde organique, qui est choisi parmi les hydroperoxydes d'alkyle et les hydroperoxydes d'arylalkyle.

**3.** Procédé selon l'une quelconque des revendications précédentes, **caractérisé en ce que** l'oxydant est utilisé en une quantité de 1 à 10 moles par mole du composé de formule II.

**4.** Procédé selon l'une quelconque des revendications précédentes, **caractérisé en ce que** le métal de transition est utilisé sous la forme d'un composé de métal de transition.

**5.** Procédé selon l'une quelconque des revendications précédentes, **caractérisé en ce que** la réaction avec l'oxydant est réalisée en présence d'un ligand complexe, qui comprend au moins un atome d'azote approprié pour la coordination avec le métal de transition.

**6.** Procédé selon l'une quelconque des revendications précédentes, **caractérisé en ce que** la réaction est réalisée dans un solvant organique ou dans un mélange de celui-ci avec de l'eau.

**7.** Procédé selon l'une quelconque des revendications précédentes, **caractérisé en ce que** la variable R dans les formules I et II signifie l'hydrogène.

**8.** Procédé selon l'une quelconque des revendications précédentes, lors de la réaction avec l'oxydant, un composé de formule générale III étant tout d'abord fabriqué :

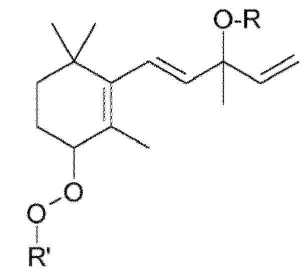

(III)

dans laquelle R représente l'hydrogène ou un groupe protecteur OH, et R' représente l'hydrogène ou le radical dérivé de l'hydroperoxyde organique, et signifie notamment hydrogène, alkyle ou arylalkyle ;
puis le composé de formule (III) étant décomposé en le composé de formule I.

**9.** Procédé selon la revendication 8, dans lequel le composé de formule III est décomposé thermiquement ou par traitement avec une base.

**10.** Composé de formule générale III

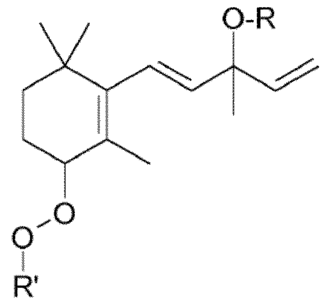

(III)

dans laquelle R représente l'hydrogène ou un groupe protecteur OH, et R' représente alkyle en $C_4$-$C_8$ tertiaire ou phényl-alkyle en $C_1$-$C_8$, le phényle étant non substitué ou portant 1, 2 ou 3 groupes alkyle en $C_1$-$C_4$.

**11.** Procédé de fabrication de sels de phosphonium de formule IV

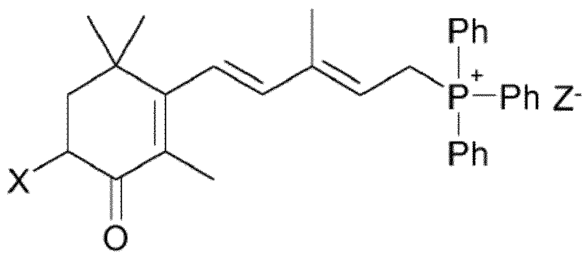

(IV)

dans laquelle X représente hydrogène, OH ou OR'', Ph représente phényle, $Z^-$ signifie un anion halogénure et R'' représente l'hydrogène ou un groupe protecteur OH, comprenant :

i) la préparation d'oxovinylionol ou d'un dérivé O-protégé de celui-ci de formule I par un procédé selon l'une quelconque des revendications 1 à 5 ou 7 ;
ii) la transformation de l'oxovinylionol ou de son dérivé O-protégé de formule I d'une manière connue en soi en un composé de formule IV.

**12.** Procédé selon la revendication 11 pour la fabrication d'un composé de phosphonium de formule IV, dans laquelle X représente OH, dans lequel l'étape ii) comprend les étapes suivantes :

a) la transformation d'un composé de formule I en un éther de silylénol de formule V

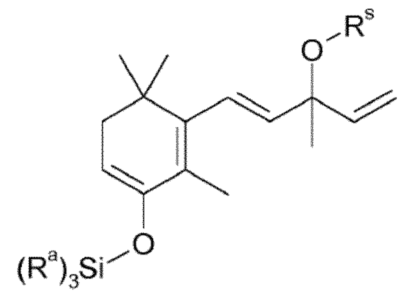

(V)

dans laquelle les $R^a$ dans le groupe $Si(R^a)_3$ peuvent être identiques ou différents, et représentent indépendamment les uns des autres alkyle en $C_1$-$C_4$, et $R^s$ représente un groupe protecteur OH clivable par un acide ;
b) la mise en réaction successive du composé de formule V avec un acide peroxocarboxylique, puis éventuellement l'hydrolyse subséquente du composé de formule VI ainsi obtenu, $R^a$ et $R^s$ ayant les significations indiquées précédemment, un composé de formule VII étant obtenu :

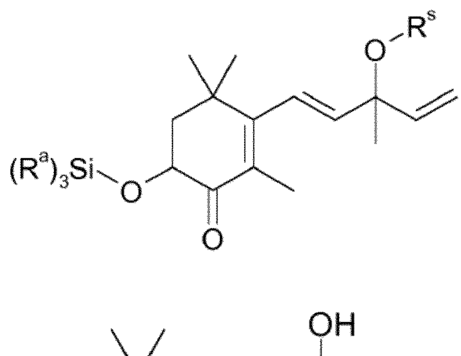

(VI)

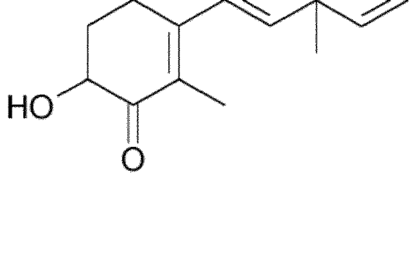

(VII)

c) la mise en réaction successive du composé de formule VII avec un agent d'halogénation, puis avec du triphénylphosphane, un composé de formule IV étant obtenu, dans laquelle X représente OH,

ou

a') la transformation d'un composé de formule I, dans laquelle R représente un groupe protecteur OH dit $R^s$ clivable par un acide, en son énolate,
b') la mise en réaction de l'énolate avec une N-arylsulfonyloxaziridine, un composé de formule VIa étant obtenu :

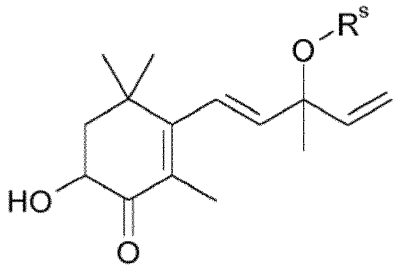
(VIa)

c) l'hydrolyse du composé de formule VIa, et la mise en réaction successive du produit d'hydrolyse avec un agent d'halogénation, puis avec du triphénylphosphane, un composé de formule IV étant obtenu, dans laquelle X représente OH.

**13.** Procédé selon l'une quelconque des revendications 11 et 12 pour la fabrication d'un composé de phosphonium de formule IV, dans laquelle X représente H, dans lequel l'étape ii) comprend l'étape suivante :
c') la mise en réaction successive du composé de formule I, dans laquelle R représente l'hydrogène, avec un halogénure d'hydrogène HZ, puis avec du triphénylphosphane, un composé de formule IV étant obtenu.

**14.** Procédé selon la revendication 11 pour la fabrication d'un sel de phosphonium de formule IVa

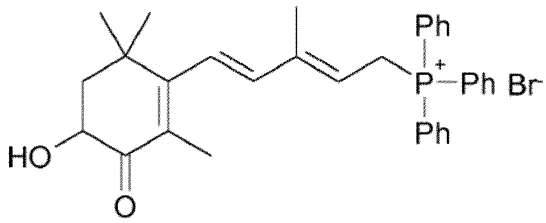
(IVa)

dans laquelle Ph représente phényle, comprenant :

a'') la préparation d'un éther de silylénol de formule V

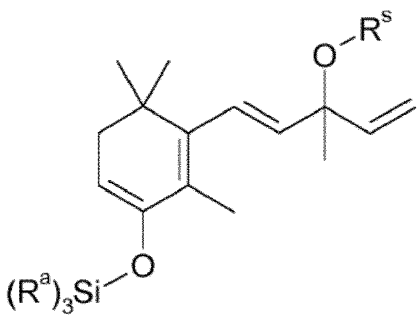
(V)

dans laquelle les $R^a$ dans le groupe $Si(R^a)_3$ peuvent être identiques ou différents, et représentent indépendamment les uns des autres alkyle en $C_1$-$C_4$, et $R^s$ représente un groupe protecteur OH clivable par un acide ;
b'') la mise en réaction successive du composé de formule V avec un acide peroxocarboxylique et la mise en réaction du produit d'oxydation avec de l'acide bromhydrique, un composé de formule VIa étant obtenu :

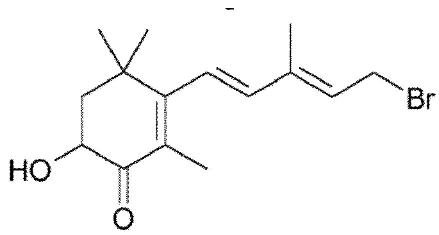

(VIa)

c') la mise en réaction du composé de formule VIa avec du triphénylphosphane, un composé de formule IVa étant obtenu.

**15.** Procédé selon la revendication 14, dans lequel la préparation du composé de formule V comprend les étapes suivantes :

i) la préparation d'oxovinylionol ou d'un dérivé O-protégé de celui-ci de formule I par un procédé selon l'une quelconque des revendications 1 à 5 ou 7 ;
ii') la transformation d'un composé de formule I en un éther de silylénol de formule V.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007072529 A
- EP 101597 A
- US 4098827 A
- WO 2008145627 A
- CN 101723769
- EP 490326 A


### In der Beschreibung aufgeführte Nicht-Patentliteratur


- *Pure Appl. Chem.,* 2002, vol. 74, 2213
- Carotenoids. *Synthesis,* vol. 2, 281-284
- **BIRKHÄUSER.** *Pure Appl. Chem.,* 1996, vol. 74 (2002), 2213
- *J. Org. Chem.,* 1982, vol. 47, 2130-2134
- **P. J. KOCIENSKI.** Protecting Groups. Georg-Thieme-Verlag, 2000
- **P.G.M. WUTS et al.** Greene's Protective Groups in Organic Synthesis. John-Wiley & Xons, 2006
- *CHEMICAL ABSTRACTS,* 1338-24-5
- *Z. Anorg. Chem,* 2004, vol. 630, 1962
- *Helv. Chim. Acta,* 1981, vol. 64, 2444